# EUROPÄISCHE PATENTSCHRIFT

(11) **EP 0 851 858 B1**
(45) Veröffentlichungstag und Bekanntmachung des Hinweises auf die Patenterteilung: **17.04.2002**
(21) Anmeldenummer: 96932485.4
(22) Anmeldetag: 09.09.1996
(51) Int. Cl.: C07D 213/61, C07D 213/26, A01N 43/40

(54) **SUBSTITUIERTE 2-PHENYLPYRIDINE ALS HERBIZIDE**
SUBSTITUTED 2-PHENYLPYRIDINES AS HERBICIDES
2-PHENYLPYRIDINES SUBSTITUEES S'UTILISANT COMME HERBICIDES

(30) Priorität: 18.09.1995 DE 19534466
(43) Veröffentlichungstag der Anmeldung: 08.07.1998
(73) Patentinhaber: BASF AKTIENGESELLSCHAFT, 67056 Ludwigshafen (DE)
(72) Erfinder: SCHÄFER, Peter, D-67308 Ottersheim (DE); HAMPRECHT, Gerhard, D-69469 Weinheim (DE); HEISTRACHER, Elisabeth, D-68159 Mannheim (DE); MENKE, Olaf, D-67317 Altleiningen (DE); ZAGAR, Cyrill, D-67061 Ludwigshafen (DE); RACK, Michael, D-69123 Heidelberg (DE); GÖTZ, Norbert, D-67547 Worms (DE); HARREUS, Albrecht, D-67063 Ludwigshafen (DE); WESTPHALEN, Karl-Otto, D-67346 Speyer (DE); WALTER, Helmut, D-67283 Obrigheim (DE); MISSLITZ, Ulf, D-67433 Neustadt (DE)
(86) Internationale Anmeldenummer: EP9603949
(87) Internationale Veröffentlichungsnummer: WO9711059

(56) Entgegenhaltungen:
- WO-A-95/02580
- DE-A- 19 500 758

## Beschreibung

Die vorliegende Erfindung betrifft neue substituierte 2-Phenylpyridine der Formel I 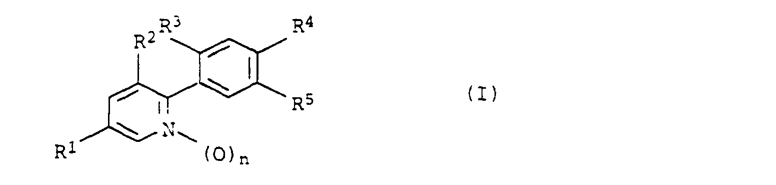 in der die Variablen folgende Bedeutungen haben:
- n: 0 oder 1;
- R¹: Halogen oder C₁-C₄-Halogenalkyl;
- R², R³: jeweils Wasserstoff oder Halogen;
- R⁴: Cyano oder Halogen;
- R⁵: -CO-O-(C₁-C₄-Alkylen)-CO-OR⁶,
-CO-O-(C₁-C₄-Alkylen)-CO-N(R⁷)R⁸,
-O-(C₁-C₄-Alkylen)-CO-O-(C₁-C₄-Alkylen)-CO-OR⁶,
-O-(C₁-C₄-Alkylen)-CO-O-(C₁-C₄-Alkylen)-CO-N(R⁷)R⁸,
-S-(C₁-C₄-Alkylen)-CO-O-(C₁-C₄-Alkylen)-CO-OR⁶ oder
-S-(C₁-C₄-Alkylen)-CO-O-(C₁-C₄-Alkylen)-CO-N(R⁷)R⁸, wobei
- R⁶: für Wasserstoff, C₁-C₄-Alkyl, C₁-C₄-Alkoxy-C₁-C₄-alkyl, C₂-C₄-Alkenyl oder C₃-C₄-Alkinyl,
- R⁷: für Wasserstoff, C₁-C₄-Alkyl, Carboxyl-C₁-C₄-alkyl, (C₁-C₄-Alkoxy)carbonyl-C₁-C₄-alkyl und
- R⁸: für Wasserstoff oder C₁-C₄-Alkyl stehen,
sowie die landwirtschaftlich brauchbaren Salze der Verbindungen I mit R⁶ = Wasserstoff.

Außerdem betrifft die Erfindung
- die Verwendung der Verbindungen I als Herbizide oder zur Desikkation und/oder Defoliaticn von Pflanzen,
- herbizide Mittel und Mittel zur Desikkation und/oder Defoliation von Pflanzen, welche die Verbindungen I als wirksame Substanzen enthalten,
- Verfahren zur Bekämpfung von unerwünschtem Pflanzenwuchs und zur Desikkation und/oder Defoliation von Pflanzen mit den Verbindungen I,
- Verfahren zur Herstellung der Verbindungen I und von herbiziden Mitteln und Mitteln zur Desikkation und/oder Defoliation von Pflanzen unter Verwendung der Verbindungen I sowie
- Zwischenprodukte der Formel IIb und ein Verfahren zu deren Herstellung.

Substituierte 2-Phenylpyridine mit herbizider Wirkung sind bereits aus der WO 95/02580, der WO 95/02590 und den älteren deutschen Anmeldungen DE-A 19 500 760, DE-A 19 500 758, DE-A 19 500 911 und DE-A 19 528 943 bekannt.

In der WO 95/02580 wird eine Vielzahl von herbidzid wirksamen 2-Phenylpyridinen beschrieben. Die Verbindungen der Formel I und deren besonders vorteilhafte Eigenschaften sind dieser Druckschrift jedoch nicht zu entnehmen.

Die herbizide Wirkung der bekannten Verbindungen bezüglich der Schadpflanzen ist jedoch nicht immer voll befriedigend. Aufgabe der vorliegenden Erfindung war es demnach, neue herbizid wirksame Verbindungen bereitzustellen, mit denen sich unerwünschte Pflanzen besser als bisher gezielt bekämpfen lassen. Die Aufgabe erstreckt sich auch auf die Bereitstellung neuer desikkant/ defoliant wirksamer Verbindungen.

Demgemäß wurden die eingangs definierten substituierten 2-Phenylpyridine der Formel I mit herbizider Wirkung sowie neue Zwischenprodukte IIb zu deren Herstellung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Des weiteren wurde gefunden, daß die Verbindungen I auch zur Defoliation/Desikkation von Pflanzenteilen geeignet sind, wofür Kulturpflanzen wie Baumwolle, Kartoffel, Raps, Sonnenblume, Sojabohne oder Ackerbohnen, insbesondere Baumwolle und Kartoffel, in Betracht kommen. Diesbezüglich wurden Mittel zur Desikkation und/ oder Defoliation von Pflanzen, Verfahren zur Herstellung dieser Mittel und Verfahren zur Desikkation und/oder Defoliation von Pflanzen mit den Verbindungen I gefunden.

Die Verbindungen der Formel I können je nach Substitutionsmuster ein oder mehrere Chiralitätszentren enthalten und liegen dann als Enantiomeren- oder Diastereomerengemische vor. Gegenstand der Erfindung sind sowohl die reinen Enantiomere oder Diastereomere als auch deren Gemische.

Die substituierten 2-Phenylpyridine I mit R⁶ = Wasserstoff können in Form ihrer landwirtschaftlich brauchbaren Salze vorliegen, wobei es auf die Art des Salzes in der Regel nicht ankommt. Im allgemeinen kommen die Salze von solchen Basen in Betracht, bei denen die herbizide Wirkung im Vergleich zu der freien Verbindung I nicht negativ beeinträchtigt ist.

Als Salze evgnen sich besonders diejenigen der Alkalimetalle, vorzugsweise Natrium- und Kaliumsalze, der Erdalkalimetalle, vorzugsweise Calcium- und Magnesiumsalze, die der Übergangsmetalle, vorzugsweise Zink- und Eisensalze, sowie Ammoniumsalze, bei denen das Ammoniumion gewünschtenfalls ein bis vier C₁-C₄-Alkyl-, Hydroxy-C₁-C₄-alkylsubstituenten und/oder einen Phenyl- oder Benzylsubstituenten tragen kann, vorzugsweise Diisopropylammonium-, Tetramethylammonium-, Tetrabutylammonium-, Trimethylbenzylammonium- und Trimethyl-(2-hydroxyethyl)-ammoniumsalze, des weiteren Phosphoniumsalze, Sulfoniumsalze wie vorzugsweise Tri-(C₁-C₄-alkyl)sulfonium-salze, und Sulfoxoniumsalze wie vorzugsweise Tri-(C₁-C₄-alkyl)sulfoxoniumsalze.

Die bei der Definition der Substituenten R¹, R⁶, R⁷ und R⁸ verwendeten Bezeichnungen Alkyl, Halogenalkyl, Alkoxy, Carboxyalkyl, Alkoxyalkyl, Alkoxycarbonylalkyl, Alkeryl und Alkinyl stellen - wie die Bedeutung Halogen - Sammelbegriffe für individuelle Aufzählungen der einzelnen Gruppenmitglieder dar. Sämtliche Alkyl-Teile können geradkettig oder verzweigt sein. Der Halogenalkyl-Rest trägt vorzugsweise ein bis funf gleiche oder verschiedene Halogenatome.

Im einzelnen stehen beispielsweise:
- Halogen für: Fluor, Chlor, Brom oder Iod;
- C₁-C₄-Alkyl für: Methyl, Ethyl, n-Propyl, 1-Methylethyl, n-Butyl, 1-Methylpropyl, 2-Methylpropyl oder 1,1-Dimethylethyl;
- C₁-C₄-Halogenalkyl für: einen C₁-C₄-Alkylrest wie vorstehend genannt, der partiell oder vollständig durch Fluor, Chlor, Brom und/oder Iod substituiert ist, also z.B. CH₂Cl, Dichlormethyl, Trichlormethyl, CH₂F, CHF₂, CF₃, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, C₂F₅, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₄-Alkoxy für: OCH₃, OC₂H₅, n-Propoxy, OCH(CH₃)₂, n-Butoxy, 1-Methylpropoxy, 2-Methylpropoxy oder 1,1-Dimethylethoxy;
- C₁-C₄-Alkoxy-C₁-C₄-alkyl für: durch C₁-C₄-Alkoxy wie Methoxy, Ethoxy, n-Propoxy, 1-Methylethoxy, n-Butoxy, 1-Methylpropoxy, 2-Methylpropoxy und 1,1-Dimethylethoxy substituiertes C₁-C₄-Alkyl, also z.B. für CH₂OCH₃, CH₂OC₂H₅, n-Propoxymethyl, (1-Methylethoxy)methyl, n-Butoxymethyl, (1-Methylpropoxy)-methyl, (2-Methylpropoxy)methyl, (1,1-Dimethylethoxy)methyl, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)propyl, 3-(n-Propoxy)propyl, 3-(1-Methylethoxy)propyl, 3-(n-3utoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(n-Propoxy)butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)-butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl, vorzugsweise CH₂OCH₃, CH₂OC₂H₅, 2-Methoxyethyl oder 2-Ethoxyethyl;
- (C₁-C₄-Alkoxy)carbonyl-C₁-C₄-alkyl für: durch (C₁-C₄-Alkoxy)carbonyl wie COOCH₃, COOC₂H₅, n-Propoxycarbonyl, COOCH(CH₃)₂, n-Butoxycarbonyl, 1-Methylpropoxycarbonyl, 2-Methylpropoxycarbonyl und COOC(CH₃)₃ substituiertes C₁-C₄-Alkyl, also z.B. für CH₂-COOCH₃, CH₂-COOC₂H₅, n-Propoxycarbonyl-methyl, CH₂-COOCH(CH₃)₂, n-Butoxycarbonylmethyl, (1-Methylpropoxycarbonyl)methyl, (2-Methylpropoxycarbonyl)-methyl, CH₂-COOC(CH₃)₃, 1-(Methoxycarbonyl)ethyl, 1-(Ethoxycarbonyl)ethyl, 1-(n-Propoxycarbonyl)ethyl, 1-(1-Methylethoxycarbonyl)ethyl, 1-(n-Butoxycarbonyl)ethyl, 2-(Methoxycarbonyl)ethyl, 2-(Ethoxycarbonyl)ethyl, 2-(n-Propoxycarbonyl)ethyl, 2-(1-Methylethoxycarbonyl)ethyl, 2-(n-Butoxycarbonyl)ethyl, 2-(1-Methylpropoxycarbonyl)ethyl, 2-(2-Methylpropoxycarbonyl)ethyl, 2-(1,1-Dimethylethoxycarbonyl)ethyl, 2-(Methoxycarbonyl)propyl, 2-(Ethoxycarbonyl)propyl, 2-(n-Propoxycarbonyl)propyl, 2-(1-Methylethoxycarbonyl)propyl, 2-(n-Butoxycarbonyl)propyl, 2-(1-Methylpropoxycarbonyl)propyl, 2-(2-Methylpropoxycarbonyl)propyl, 2-(1,1-Dimethylethoxycarbonyl)propyl, 3-(Methoxycarbonyl)propyl, 3-(Ethoxycarbonyl)propyl, 3-(n-Propoxycarbonyl)propyl, 3-(1-Methylethoxycarbonyl)-propyl, 3-(n-Butoxycarbonyl)propyl, 3-(1-Methylpropoxycarbonyl)propyl, 3-(2-Methylpropoxycarbonyl)propyl, 3-(1,1-Dimethylethoxycarbonyl)propyl, 2-(Methoxycarbonyl)-butyl, 2-(Ethoxycarboryl)butyl, 2-(n-Propoxycarbonyl)butyl, 2-(1-Methylethoxycarbonyl)butyl, 2-(n-Butoxycarbonyl)butyl, 2-(1-Methylpropoxycarbonyl)butyl, 2-(2-Methylpropoxycarbonyl)butyl, 2-(1,1-Dimethylethoxycarbonyl)butyl, 3-(Methoxycarbonyl)butyl, 3-(Ethoxycarbonyl)butyl, 3-(n-Propoxycarbonyl)butyl, 3-(1-Methylethoxycarbonyl)butyl, 3-(n-Butoxycarbonyl)butyl, 3-(1-Methylpropoxycarbonyl)butyl, 3-(2-Methylpropoxycarbonyl)butyl, 3-(1,1-Dimethylethoxycarbonyl)butyl, 4-(Methoxycarbonyl)butyl, 4-(Ethoxycarbonyl)butyl, 4-(n-Propoxycarbonyl)butyl, 4-(1-Methylethoxycarbonyl)butyl, 4-(n-Butoxycarbonyl)butyl, 4-(1-Methylpropoxycarbonyl)butyl, 4-(2-Methylpropoxycarbonyl)butyl oder 4-(1,1-Dimethylethoxycarbonyl)butyl, vorzugsweise CH₂-COOCH₃, CH₂-COOC₂H₅, 1-(Methoxycarbonyl)ethyl oder 1-(Ethoxycarbonyl)ethyl;
- Carboxyl-C₁-C₄-alkyl für Carboxylmethyl, 1-Carboxylethyl, 2-Carboxylethyl, 1-Carboxylprop-1-yl, 2-Carboxylprop-1-yl, 3-Carboxylprop-1-yl, 1-Carboxylbut-1-yl, 2-Carboxylbut-1-yl, 3-Carboxylbut-1-yl, 4-Carboxylbut-1-yl, 1-Carboxylbut-2-yl, 2-Carboxylbut-2-yl, 3-Carboxylbut-2-yl, 3-Carboxylbut-2-yl, 4-Carboxylbut-2-yl, 1-(Carboxylmethyl)eth-1-yl, 1-(Carboxylmethyl)-1-(methyl)-eth-1-yl oder 1-(Carboxylmethyl)prop-1-yl, vorzugsweise für Carboxylmethyl oder 1-Carboxylethyl;
- C₂-C₄-Alkenyl für: Vinyl, Prop-1-en-1-yl, Allyl, 1-Methylethenyl, 1-Buten-1-yl, 1-Buten-2-yl, 1-Buten-3-yl, 2-Buten-1-yl, 1-Methylprop-1-en-1-yl, 2-Methylprop-1-en-1-yl, 1-Methylprop-2-en-1-yl und 2-Methylprop-2-en-1-yl, vorzugsweise für Allyl oder 2-Buten-1-yl;
- C₃-/C₄-Alkinyl für: Prop-1-in-1-yl, Prop-2-in-1-yl, n-But-1-in-1-yl, n-But-1-in-3-yl, n-But-1-in-4-yl oder n-But-2-in-1-yl, vorzugsweise für Prop-2-in-1-yl.

C₁-C₄-Alkylen steht z.B. für Methylen, 1,1-Ethylen, 1,2-Ethylen, 1,1-Propylen, 1,2-Propylen, 1,3-Propylen, 2,2-Propylen, 1,1-Butylen, 1,2-Butylen, 1,3-Butylen, 1,4-Butylen, 2,2-Butylen, 2,3-Butylen, 2-Methyl-1,1-propylen, 2-Methyl-1,2-propylen oder 2-Mechyl-1,3-propylen, vorzugsweise für Methylen, 1,1-Ethylen oder 2,2-Propylen.

Im Hinblick auf die Verwendung der erfindungsgemäßen substituierten 2-Phenylpyridine I als Herbizide und/oder als desikkant/ defoliant wirksame Verbindungen haben die Variablen vorzugsweise folgende Bedeutungen, und zwar jeweils für sich allein oder in Kombination:
- n: Null;
- R¹: Chlor oder Trifluormethyl;
- R²: Chlor;
- R³: Wasserstoff, Fluor oder Chlor;
- R⁴: Cyano oder Chlor;
- R⁵: -CO-O-(C₁-C₄-Alkylen)-CO-OR⁶, -CO-O-(C₁-C₄-Alkylen)-CO-N(R⁷)R⁸,
-O-(C₁-C₄-Alkylen)-CO-O-(C₁-C₄-Alkylen)-CO-OR⁶ oder
-O-(C₁-C₄-Alkylen)-CO-O-(C₁-C₄-Alkylen)-CO-N(R⁷)R⁸, wobei
- R⁶: für C₁-C₄-Alkyl, C₂-C₄-Alkenyl oder C₃-C₄-Alkinyl,
- R⁷: für C₁-C₄-Alkyl oder (C₁-C₄-Alkoxy)carbonyl-C₁-C₄-alkyl und
- R⁸: für Wasserstoff oder C₁-C₄₋Alkyl stehen.

Besonders bevorzugt sind die substituierten 2-Phenylpyridine Ia (≙ I mit n = Null, R² und R⁴ = Chlor), insbesondere die in der folgenden Tabelle 1 aufgeführten Verbindungen:

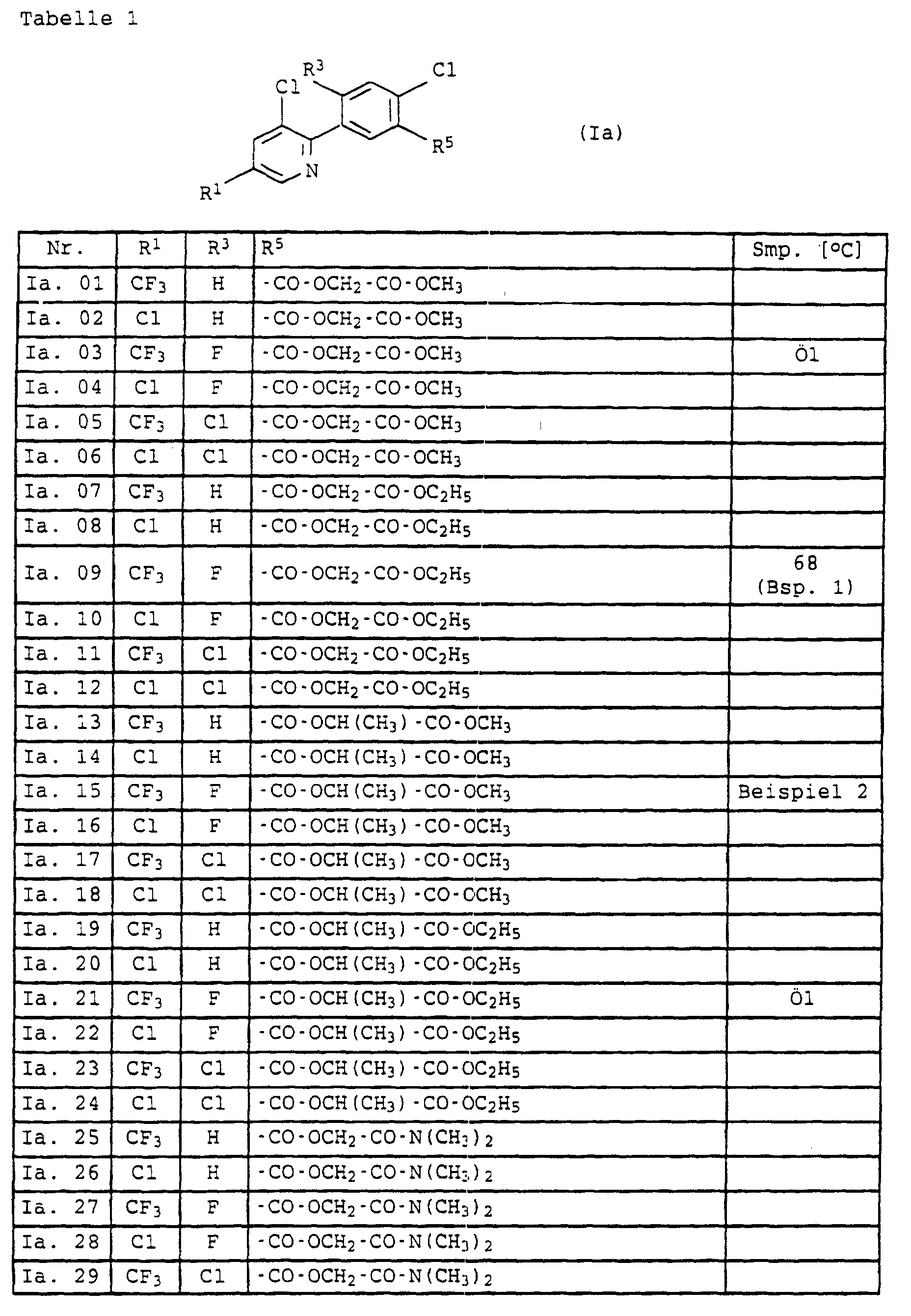

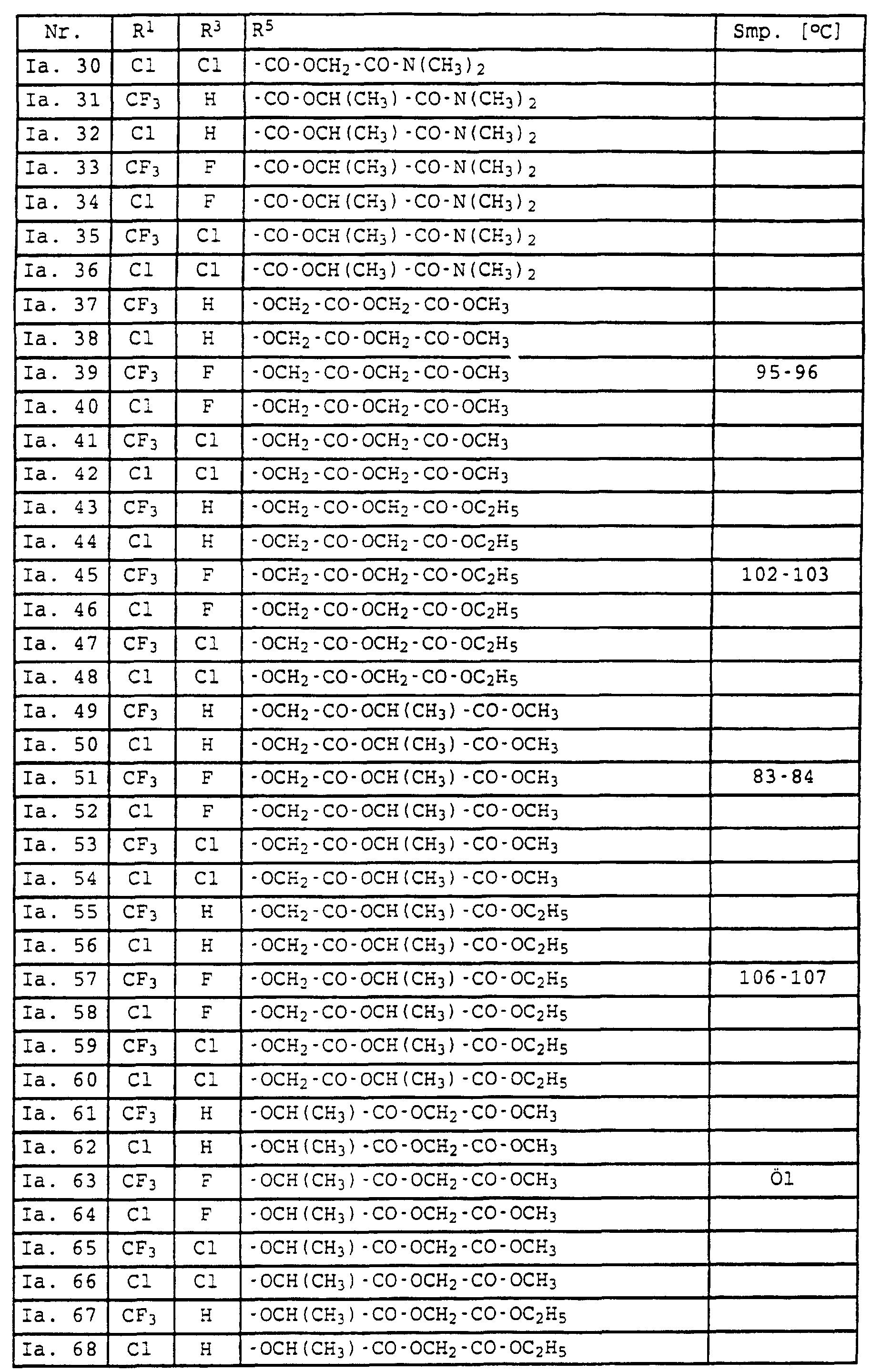

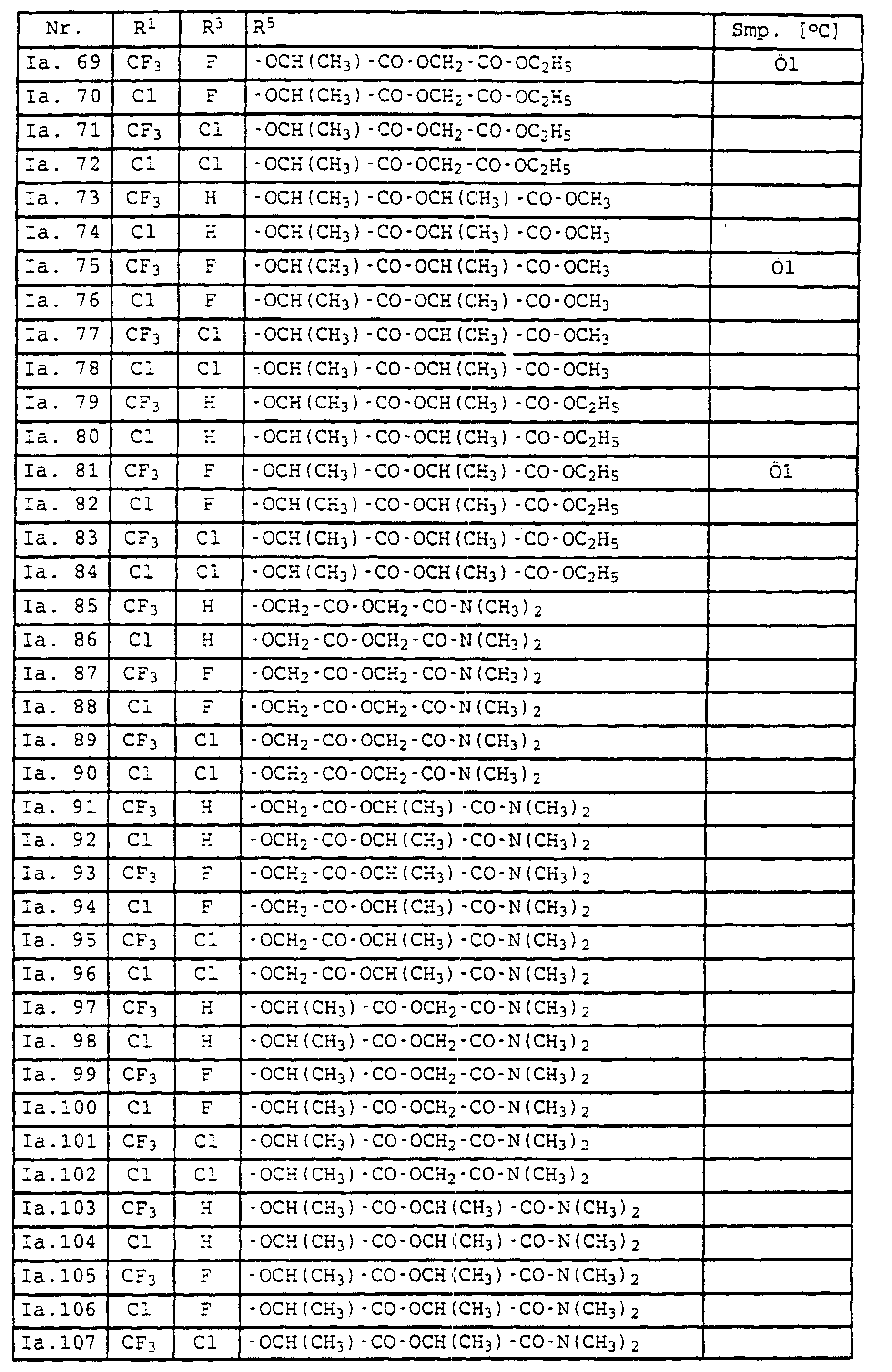

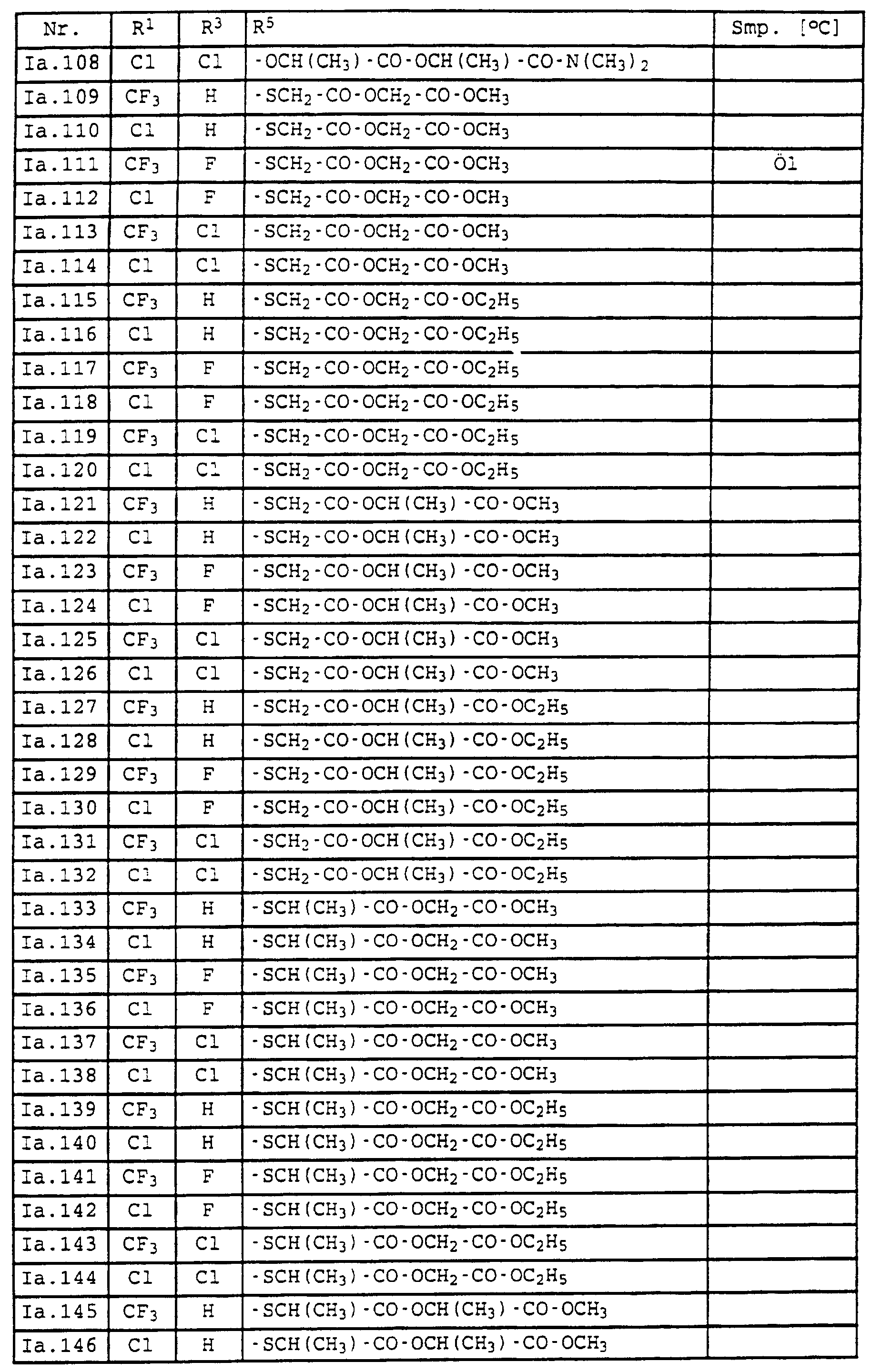

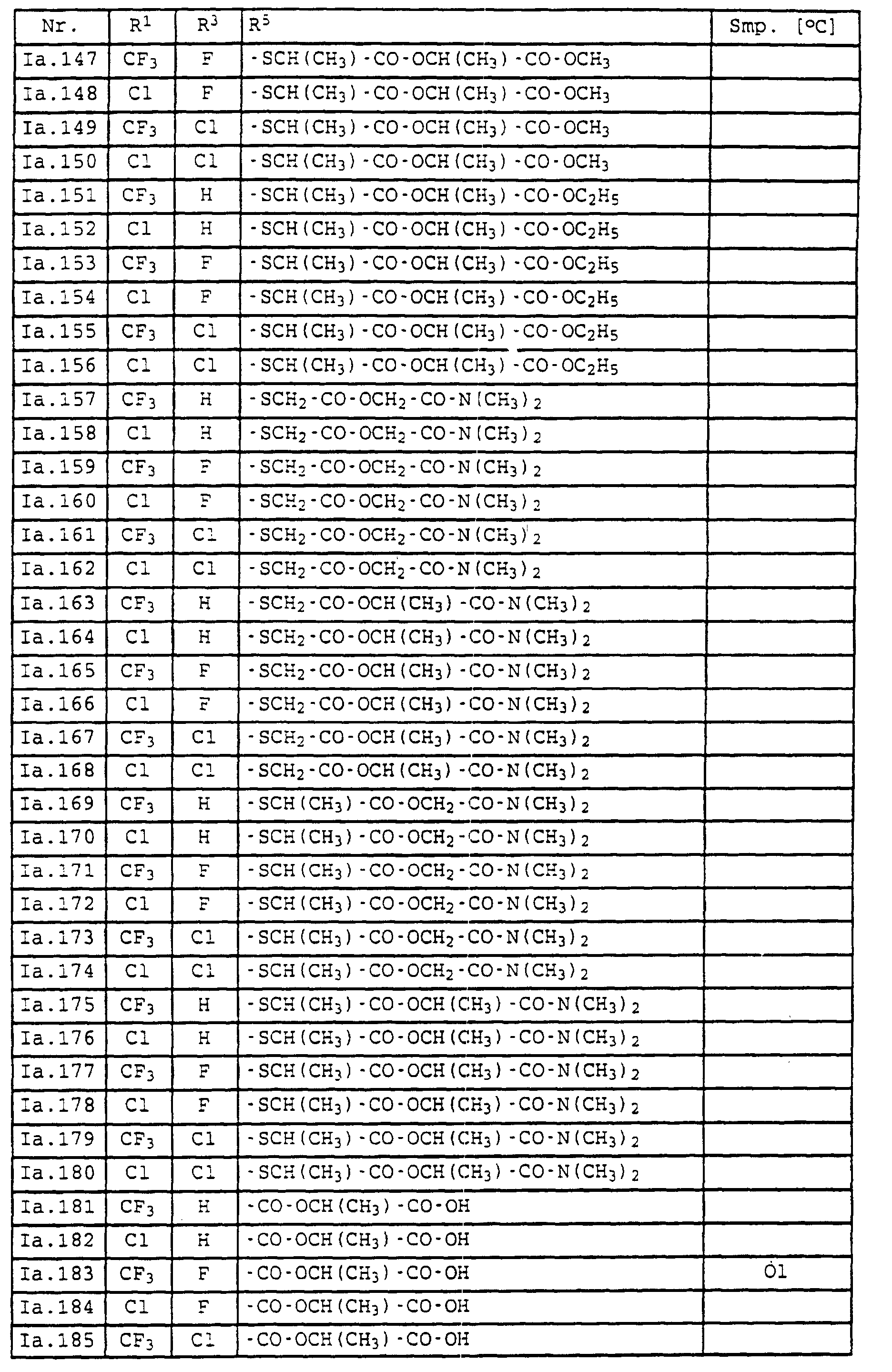

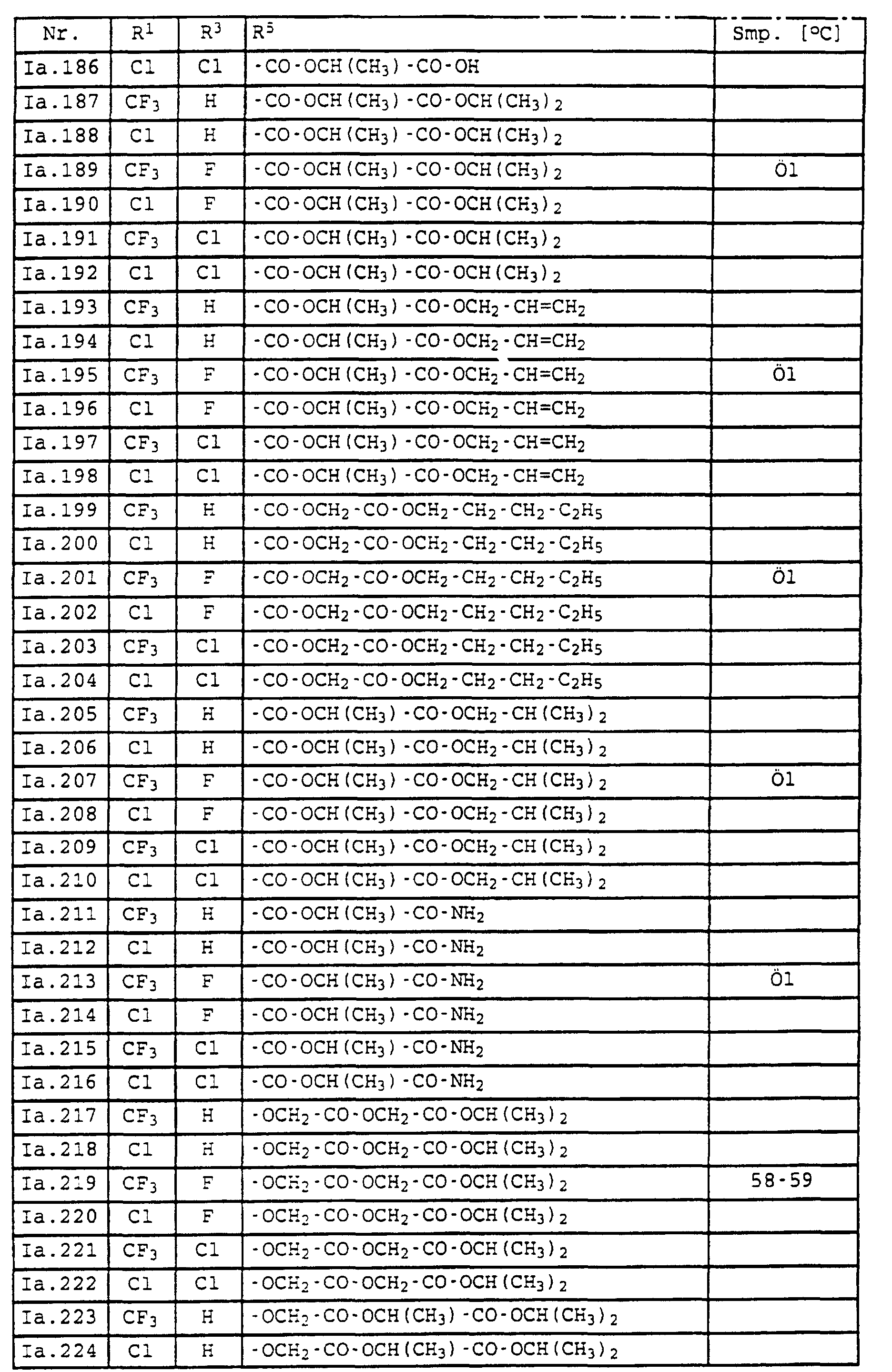

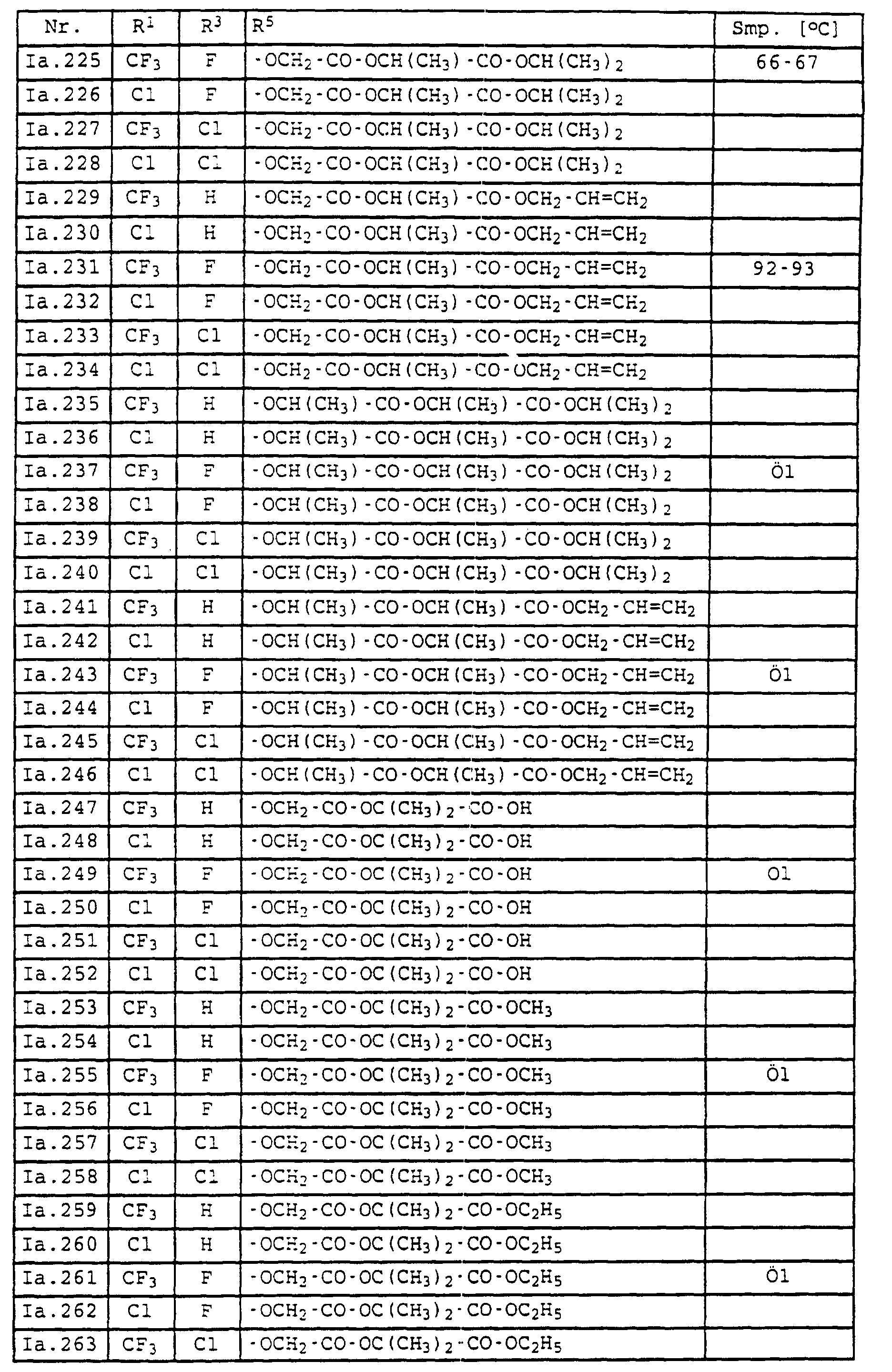

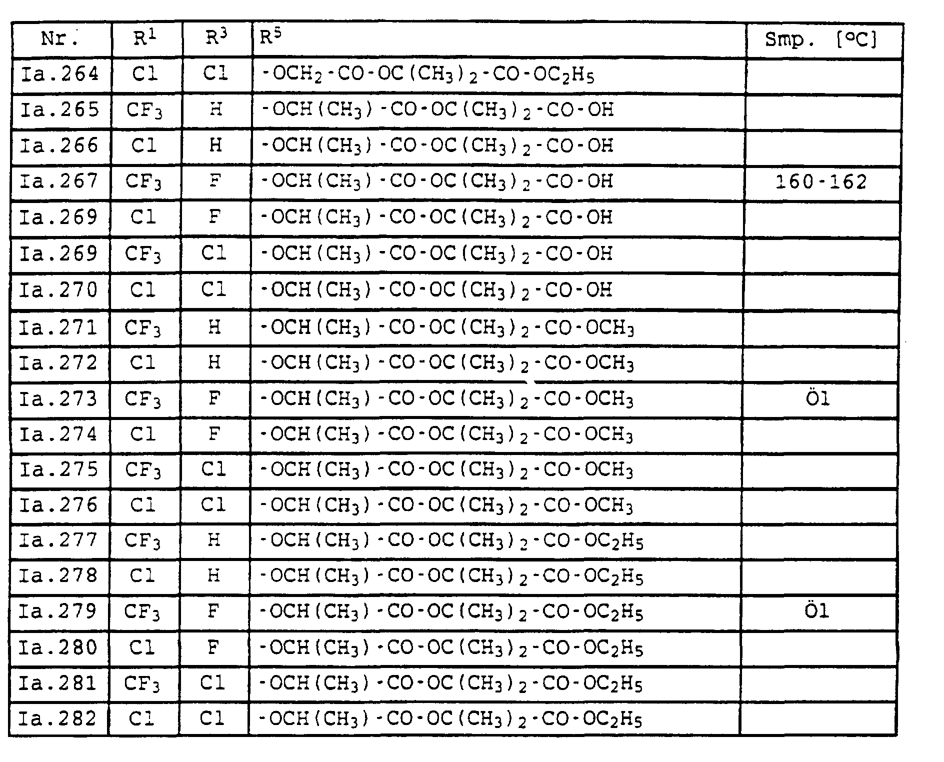

Besonders bevorzugt sind weiterhin die substituierten 2-Phenylpyridine Ib, insbesondere die Verbindungen Ib.01 bis Ib.282, die sich von den entsprechenden Verbindungen Ia.01 bis Ia.282 lediglich dadurch unterscheiden, daß R⁴ für Cyano steht:

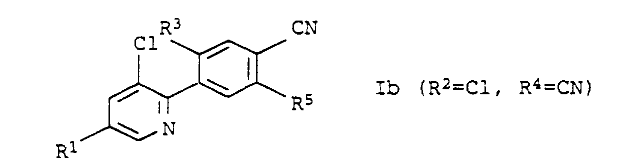

Die substituierten 2-Phenylpyridine der Formel I sind auf verschiedene Weise erhältlich, beispielsweise nach einem der folgenden Verfahren:

### Verfahren A)

Umsetzung von Säurechloriden IIa oder IIc mit Hydroxycarbonsäure(derivate)n IIIa oder IIIb oder von Säurechloriden IIb mit Alkoholen IIIc oder Aminen IIId in Gegenwart einer Base (vgl. z.B. K. Furuta et al., Org. Synth. 72, 86 (1993) und H. Henecka in Houben-Weyl, Methoden der Organischen Chemie, Bd. VIII, 4. Auflage Stuttgart 1952, Seiten 463ff.):

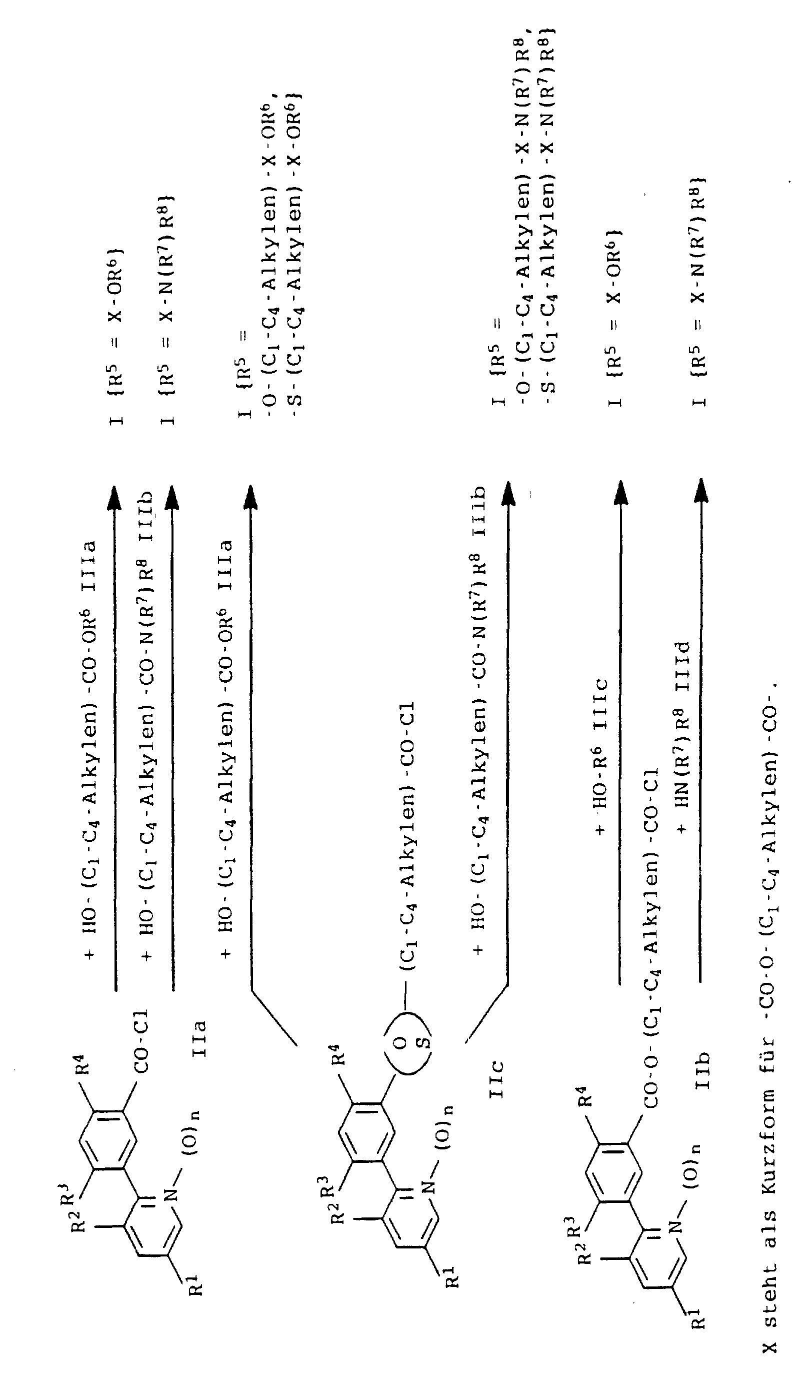

Üblicherweise arbeitet man in einem inerten Lösungs- oder Verdünnungsmittel, insbesondere in einem halogenierten Kohlenwasserstoff wie Dichlormethan, Chloroform, 1,2-Dichlorethan und Tetrachlorkohlenstoff.

Als Basen kommen z.B. Alkalimetall(hydrogen)carbonate wie Natriumhydrogencarbonat und Natriumcarbonat, ferner Stickstoffbasen wie Pyridin, 4-Dimethylaminopyridin und Triethylamin in Betracht.

Die Reaktionstemperatur liegt normalerweise bei 0 bis 100°C.

Üblicherweise werden die Komponenten in etwa stöchiometrischen Mengen eingesetzt, jedoch kann ein Überschuß einer der Komponenten, Z.B. im Hinblick auf eine möglichst vollständige Umsetzung der anderen Komponente, vorteilhaft sein.

Die Säurechloride der Formel IIa und IIc sind aus der älteren deutschen Anmeldung DE-A 19 500 758 bekannt. Die Säurechloride IIb sind neu und zweckmäßigerweise durch Umsetzung von IIa mit Hydroxycarbonsäuren HO-(C₁-C₄-Alkylen)-COOH (IV) oder deren Salzen und anschließender Chlorierung der erhaltenen Verfahrensprodukte (V) zugänglich:

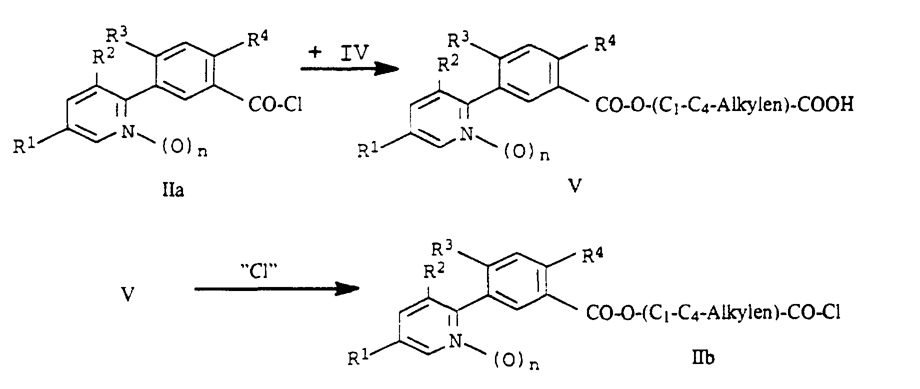

Brauchbare Salze von IV sind vor allem die Alkalimetallsalze, insbesondere die Natrium- und Kaliumsalze.

Die Chlorierung kann entweder ohne Lösungsmittel in einem Überschuß des Halogenierungsmittels oder in einem inerten Lösungsoder Verdünnungsmittel, insbesondere in einem aprotischen Lösungsmittel, z.B. in Diethylether, Benzol oder in Schwefelkohlenstoff, erfolgen.

Als Chlorierungsmittel kommen z.B. Thionylchlorid, Oxalylchlorid, Phosphortrichlorid, Phosphorpentachlorid, Phosphoroxichlorid, Phosgen, Diphosgen oder Triphosgen in Betracht.

Weitere Angaben zur Durchführung derartiger Chlorierungsreaktionen sind in der folgenden Literatur zu finden, auf die beispielhaft verwiesen wird:
- A.J. Meyers und M.E. Flanagan, Org. Synth. 71, 107 (1992);
- H.J. Scheifele Jr. und D.F. DeTar, Org. Synth. Coll. Vol. IV, Seite 34 (1963);
- G.H. Coleman et al., Org. Synth. Coll. Vol. III, Seite 712 (1955);
- H. Henecka in Houben-Weyl, Methoden der Organischen Chemie, Bd. VIII, 4. Auflage Stuttgart 1952, Seiten 463ff.

Die den Säurechloriden III entsprechenden Carbonsäuren sind z.B. aus der DE-A 43 23 916 bekannt oder auf die dort beschriebene Weise erhältlich.

### Verfahren B)

Oxidation von substituierten 2-Phenylpyridinen der Formel I, bei denen n Null bedeutet und der Substituent R⁵ keine Schwefelbrücke enthält, auf an sich bekannte Weise {vgl. z.B. A. Albini & S. Pietra, Heterocyclic N-Oxides, CRC-Press Inc., Boca Raton, USA 1991; H.S. Mosher et al., Org. Synth. Coll. Vol. IV 1963, Seite 828; E.C. Taylor et al., Org. Synth. Coll. Vol. IV 1963, Seite 704; T.W. Bell et. al., Org. Synth. 69, Seite 226 (1990)}: 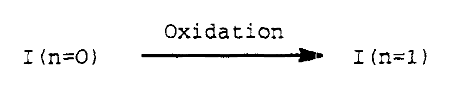
Unter den zur Oxidation des Pyridinrings üblichen Oxidationsmitteln sei beispielhaft auf Peressigsäure, Trifluorperessigsäure, Perbenzoesäure, m-Chlorperbenzoesäure, Monopermaleinsäure, Magnesiummonoperphthalat, Natriumperborat, Oxone® (enthält Peroxidisulfat), Perwolframsäure und Wasserstoffperoxid verwiesen.
Geeignete Lösungsmittel sind z.B. Wasser, Schwefelsäure, Carbonsäuren wie Essigsäure und Trifluoressigsäure sowie halogenierte Kohlenwasserstoffe wie Dichlormethan und Chloroform.
Normalerweise gelingt die Oxidation bei Temperaturen von 0°C bis Siedetemperatur des Reaktionsgemisches.
Das Oxidationsmittel wird normalerweise in mindestens äquimolaren Mengen, bezogen auf die Ausgangsverbindung, eingesetzt. Im allgemeinen hat sich ein Überschuß an Oxidationsmittel als besonders vorteilhaft erwiesen.

### Verfahren C)

Umsetzung von 3-Pyridylphenolen der Formel VI mit Elektrophilen der Formel VII oder VIII in Gegenwart einer Base: 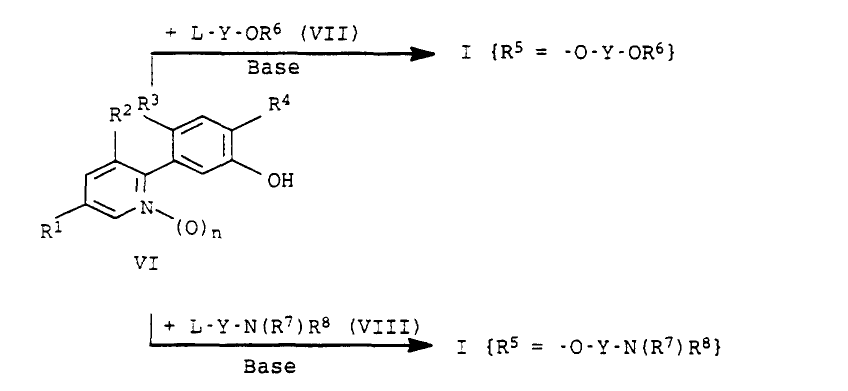
Y steht für die Kette -(C₁-C₄-Alkylen)-CO-O-(C₁-C₄-Alkylen)-CO-.
L steht für Chlor, Brom, Jod, Methylsulfonyloxy, Trifluormethylsulfonyloxy, Phenylsulfonyloxy oder p-Tolylsulfonyloxy.

In der Regel arbeitet man in einem inerten Lösungs- oder Verdünnungsmittel, das vorzugsweise aprotisch ist, also z.B. in N,N-Dimethylformamid, Dimethylsulfoxid, Aceton, N-Methylpyrrolidon, Acetonitril oder in einem Ether wie Diethylether, Tetrahydrofuran und 1,4-Dioxan.

Brauchbare Basen sind beispielsweise Alkalimetallcarbonate und -hydrogencarbonate wie Natriumhydrogencarbonat, Kaliumhydrogencarbonat, Natriumcarbonat und Kaliumcarbonat, Alkalimetallalkoholate wie Natriummethanolat und Kalium-tert.-butanolat, Alkalimetallhydroxide wie Natriumhydroxid, und Alkalimetallhydride wie Natriumhydrid.

Weitere Angaben zur Durchführung derartiger Alkylierungsreaktionen sind beispielsweise in der folgenden Literatur zu finden:
* zur Alkylierung von Phenolen mit α-Carbonylsulfonaten:
   - U. Burkard und F. Effenberger, Chem. Ber. 119, 1594 (1986);
   - J. Bierdermann et al., J. Med. Chem. 29, 1183 (1986);
   - R.B. Rogers et al., U.S. 4,725,683.

** zur Alkylierung von Phenolen mit α-Halogenestern:
   - R. Aneja et al., Tetrahedron 2, 203 (1958);
   - EP-A 380 043;
   - C.R. Edwards et al., J. Heterocycl. Chem. 24, 495 (1987);
   - C.P. Phadke et al., Synthesis 5, 413 (1986);
   - K.G. Watson, U.S. 4,837,355;
   - V. Elango et al., U.S. 4,908,476;
   - G. Schlegel et al., U.S. 4,978,774;
   - U. Burkard und F. Effenberger, Chem. Ber. 119, 1594 (1986);
   - H. Sugihara et al., Chem. And Pharm. Bull. 35, 1919 (1987);
   - S. Fujinawa et al., U.S. 4,625,053.

Die Elektrophile VII und VIII sind bekannt oder auf an sich bekannte weise erhältlich {vgl. hierzu z.B. EP-A 537 838; E.K. Euranto, Suom. Kemistilehti B 43(9), 324-327 (1970); DE-A 43 20 396; JP 04/001 190; DE-A 25 01 448; U.S. 4,033,938; M. Franck-Neumann et al., Synlett 10, 637-640 (1990); J.H. Clark et al., J. Chem. Soc., Dalton Trans. 20, 2129-2134 (1975) und FR 2 459 221).

### Verfahren D)

Umsetzung von 3-Pyridylthiophenolen der Formel IX mit Elektrophilen VII oder VIII in Gegenwart einer Base:

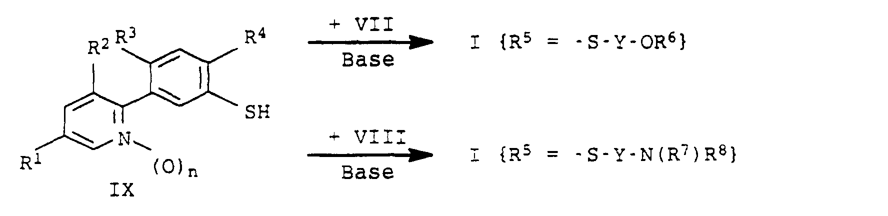

Bezüglich der Definition von Y und L sowie geeigneter Lösungs-/Verdünnungsmittel und Basen gelten die Angaben für Verfahren C).

weitere Angaben zur Durchführung derartiger Akylierungsreaktionen sind beispielsweise in der folgenden Literatur zu finden:
* zur Alkylierung von Thiophenolen mit α-Carbonylsulfonaten:
   - U. Burkard und F. Effenberger, Chem. Ber. 119, 1594 (1986);

** zur Alkylierung von Thiophenolen mit α-Halogenestern:
   - M.B. Floyd, U.S. 4,983,753;
   - E. Campaigne und A.R. McLaughlin, J. Heterocycl. Chem. 20, 623 (1983);
   - J. Durman et al., J. Chem. Soc. Perkin Trans., 1939 (1986);
   - M. Kawada et al., Chem. Pharm. Bull. 34, 1939 (1986);
   - H. Sugihara et al., Chem. And Pharm. Bull. 35, 1919 (1987).

Sofern nicht anders angegeben werden alle vorstehend beschriebenen Verfahren zweckmäßigerweise bei Atmosphärendruck oder unter dem Eigendruck des jeweiligen Reaktionsgemisches vorgenommen.

Normalerweise sind die substituierten 2-Phenylpyridine I nach nach einem der vorstehend genannten Syntheseverfahren herstellbar. Aus wirtschaftlichen oder verfahrenstechnischen Gründen kann es jedoch zweckmäßiger sein, einige Verbindungen I aus ähnlichen 2-Phenylpyridinen, die sich jedoch in der Bedeutung eines Restes unterscheiden, herzustellen.

Die Aufarbeitung der Reaktionsgemische erfolgt in der Regel nach an sich bekannten Methoden, beispielsweise
durch Verdünnen äer Reaktionslösung mit Wasser und anschließender Isolierung des Produktes mittels Filtration, Kristallisation oder Lösungsmittelextraktion, oder
durch Entfernen des Lösungsmittels, Verteilen des Rückstandes in einem Gemisch aus Wasser und einem geeigneten organischen Lösungsmittel und Aufarbeiten der organischen Phase auf das Produkt hin.

Die substituierten 2-Phenylpyridine der Formel I können ein oder mehrere Chiralitätszentren enthalten und fallen dann üblicherweise als Enantiomeren- oder Diastereomerengemische an. Die Mischungen können gewünschtenfalls nach den hierfür üblichen Methoden wie Kristallisation oder Chromatographie, auch an einem optisch aktiven Adsorbat, in die weitgehend reinen Isomeren getrennt werden. Reine optisch aktive Isomere lassen sich beispielsweise auch aus entsprechenden optisch aktiven Ausgangsmaterialien herstellen.

Diejenigen substituierten 2-Phenylpyridine I mit R⁶ = Wasserstoff lassen sich auf an sich bekannte Weise in ihre Salze, vorzugsweise in ihre Alkalimetallsalze, überführen.

Salze von I, deren Metallinn kein Alkalimetallion ist, können durch Umsalzen des entsprechenden Alkalimetallsalzes in üblicher Weise hergestellt werden, ebenso Ammonium-, Phosphonium-, Sulfonium- und Sulfoxoniumsalze mittels Ammoniak, Phosphonium-, Sulfonium- oder Sulfoxoniumhydroxiden.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Cameilia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus sind die Verbindungen I auch in Kulturen, die durch Züchtung einschließlich gentechnischer Methoden gegen die Wirkung von Herbiziden tolerant sind, verwendbar.

Des weiteren eignen sich die substituierten 2-Phenylpyridine I auch zur Desikkation und/oder Defoliation von Pflanzen.

Als Desikkantien eignen sie sich insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- oder Blatt- und Sproßteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Faserqualität nach der Ernte.

Die Verbindungen I bzw. die sie enthaltenden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe für die Herstellung von direkt versprühbaren Lösungen, Emulsionen, Pasten oder Öldispersionen kommen im wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Im allgemeinen enthalten die Formulierungen etwa von 0,001 bis 98 Gew.-%, vorzugsweise 0,01 und 95 Gew.-%, mindestens eines Wirkstoffs. Die Wirkstoffe werden dabei in einer Reinheit von 90 % bis 100 %, vorzugsweise 95 % bis 100 % (nach NMR-Spektrum) eingesetzt.

Die folgenden Formulierungsbeispiele verdeutlichen die Herstellung solcher Zubereitungen:
I. 20 Gewichtsteile der Verbindung Nr. Ia.09 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung Nr. Ia.15 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile des Wirkstoffs Nr. Ia.39 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew. -% des Wirkstoffs enthält.
IV. 20 Gewichtsteile des Wirkstoffs Nr. Ia.51 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew. -% des Wirkstoffs enthält.
V. 3 Gewichtsteile des Wirkstoffs Nr. Ia.57 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. Ia.81 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil des Wirkstoffs Nr. Ia.195 wird in einer Mischung gelöst, die aus 70 Gewichtsteilen Cyclohexanon, 20 Gewichtsteilen ethoxyliertem Isooctylphenol und 10 Gewichtsteilen ethoxyliertem Ricinusöl besteht. Man erhält ein stabiles Emulsionskonzentrat, das mit Wasser auf die gewünschte Wirkstoffkonzentration verdünnt werden kann.
VIII.1 Gewichtsteil des Wirkstoffs Nr. Ia.261 wird in einer Mischung gelöst, die aus 80 Gewichtsteilen Cyclohexanon und 20 Gewichtsteilen Wettol® EM 31 (= nichtionischer Emulgator auf der Basis von ethoxyliertem Rizinusöl; BASF AG) besteht. Man erhält ein stabiles Emulsionskonzentrat.

Die Applikation der Wirkstoffe I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I betragen je nach Bekämpfungsziel, Jahreszeit, Zielpflanzen und Wachstumsstadium 0,001 bis 3,0, vorzugsweise 0,01 bis 1 kg/ha aktive Substanz (a.S.).

Zur Verbreiterung des Wirkungsspektrums und zur Erzielung synergistischer Effekte können die substituierten 2-Phenylpyridine I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiele

### Beispiel 1

### Verbindung Nr. Ia.09 in Tabelle 1

3,0 g 3-Chlor-2-(5-carboxy-4-chlor-2-fluorphenyl)-5-trifluormethylpyridin wurden 2,5 Stunden in 20 ml Thionylchlorid auf Rückflußtemperatur erhitzt. Dann destillierte man das überschüssige Thionylchlorid bei vermindertem Druck ab, wonach der Rückstand in 25 ml wasserfreiem Methylenchlorid gelöst wurde. Nach Zugabe von 0,8 g Pyridin und 5,0 g Glykolsäureethylester wurde die Reaktionsmischung noch 7,5 Stunden auf Rückflußtemperatur erhitzt und dann etwa 15 Stunden bei 23°C gerührt.

Zur Aufarbeitung wurde der Reaktionsansatz zunächst bei vermindertem Druck eingeeng. Nach Aufnehmen des Rückstandes mit 50 ml Methylenchlorid extrahierte man nacheinander mit 50 ml 0,1-molarer Salzsäure und 50 ml Wasser. Anschließend wurde die organische Phase über Natriumsulfat getrocknet und dann eingeengt. Die Reinigung des Rückstandes erfolgte chromatographisch an Kieselgel (Laufmittel: Cyclohexan/Essigester = 10:1). Ausbeute: 2,3 g farbloser Kristalle; Schmelzpunkt: 68°C.
¹H-NMR (200 MHz, in CDCl₃): δ [ppm] = 1.30 (t,3H), 4.27 (q,2H), 4.85 (s,2H), 7.37 (d,1H), 8.08 (s,1H), 8.20 (d,1H), 8.90 (s,1H).

### Beispiel 2

### Verbindung Nr. Ia.15 in Tabelle 1

Analog Beispiel 1 erhielt man unter Verwendung von 3,0 g 3-Chlor-2-(5-carboxy-4-chlor-2-fluorphenyl)-5-trifluormethylpyridin, 1,35 g Pyridin und 1,77 g (s)-Milchsäuremethylester 1,9 g eines farblosen Öls.
¹H-NMR (200 MHz, in CDCl₃): δ [ppm] = 1.63 (d,3H), 3.78 (s,3H), 5.35 (q,1H), 7.37 (d,1H), 8.08 (s,1H), 8.17 (d,1H), 8.88 (s,1H).

### Anwendungsbeispiele (herbizide Wirksamkeit)

Die herbizide Wirkung der substituierten 2-Phenylpyridine I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,98 oder 0,49 g/ha a.S. (aktive Substanz).

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Galium aparine | Kettenlabkraut | catchweed bedstraw |
| Ipomoea subspecies | Prunkwindearten | morningglory |
| Polygonum persicaria | Flohknöterich | ladysthumb |
| Sinapis alba | Weißer Senf | white mustard |

Bei einer Aufwandmenge von 0,98 oder 0,49 g/ha a.S. zeigte die Verbindung Nr. Ia.15 im Nachauflaufverfahren eine gute bis sehr gute Wirkung gegen die o.g. Pflanzen.

Die aus der WO 95/02580 bekannte (dort Nr. 1.599) und zum Vergleich ebenfalls getestete Verbindung 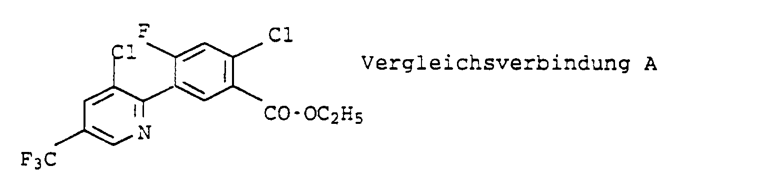 war dagegen weniger wirksam.

### Anwendungsbeispiele (desikkative/defoliante Wirksamkeit)

Als Testpflanzen dienten junge, 4-blättrige (ohne Keimblätter) Baumwollpflanzen, die unter Gewächshausbedingungen angezogen wurden (rel. Luftfeuchtigkeit 50 bis 70 %; Tag-/Nachttemperatur 27/20°C).

Die jungen Baumwollpflanzen wurden tropfnaß mit wässrigen Aufbereitungen der Wirkstoffe (unter Zusatz von 0,15 Gew.-% des Fettalkoholalkoxylats Plurafac® LF 700 ¹⁾, bezogen auf die Spritzbrühe) blattbehandelt. Die ausgebrachte Wassermenge betrug umgerechnet 1000 l/ha. Nach 13 Tagen wurde die Anzahl der abgeworfenen Blätter und der Grad der Entblätterung in % bestimmt.
¹⁾ ein schaumarmes, nichtionisches Tensid der BASF AG

Bei den unbehandelten Kontrollpflanzen trat kein Blattfall auf.

## Patentansprüche

1. Substituierte 2-Phenylpyridine der allgemeinen Formel I 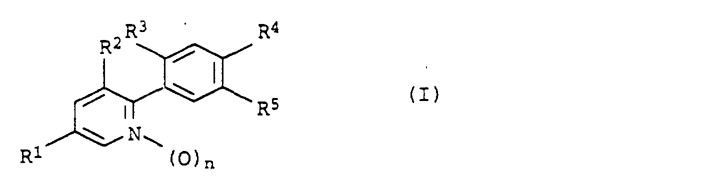 in der die Variablen folgende Bedeutungen haben:
n 0 oder 1;
R¹ Halogen oder C₁-C₄-Halogenalkyl;
R², R³ jeweils Wasserstoff oder Haiogen;
R⁴ Cyano oder Halogen;
R⁵ -CO-O-(C₁-C₄-Alkylen)-CO-OR⁶,
-CO-O-(C₁-C₄-Alkylen)-CO-N(R⁷)R⁸,
-O-(C₁-C₄-Alkylen)-CO-O-(C₁-C₄-Alkylen)-CO-OR⁶,
-O-(C₁-C₄-Alkylen)-CO-O-(C₁-C₄-Alkylen)-CO-N(R⁷)R⁸,
-S-(C₁-C₄-Alkylen)-CO-O-(C₁-C₄-Alkylen)-CO-OR⁶ oder
-S-(C₁-C₄-Alkylen)-CO-O-(C₁-C₄-Alkylen)-CO-N(R⁷)R⁸, wobei
R⁶ für Wasserstoff, C₁-C₄-Alkyl, C₁-C₄-Alkoxy-C₁-C₄-alkyl, C₂-C₄-Alkenyl oder C₃-C₄-Alkinyl,
R⁷ für Wasserstoff, C₁-C₄-Alkyl, Carboxyl-C₁-C₄-alkyl, (C₁-C₄-Alkoxy)carbonyl-C₁-C₄-alkyl und
R⁸ für Wasserstoff oder C₁-C₄-Alkyl stehen,
sowie die landwirtschaftlich brauchbaren Salze der Verbindungen I mit R⁶ = Wasserstoff.

2. Substituierte 2-Phenylpyridine der Formel I nach Anspruch 1, wobei die Variablen folgende Bedeutungen haben:
n null,
R¹ Chlor oder Trifluormethyl,
R² Chlor,
R³ Wasserstoff, Fluor oder Chlor,
R⁴ Cyano oder Chlor,
R⁵ -CO-O-(C₁-C₄-Alkylen)-CO-OR⁶,
-CO-O-(C₁-C₄-Alkylen)-CO-N(R⁷)R⁸,
-O-(C₁-C₄-Alkylen)-CO-O(C₁-C₄-Alkylen)-CO-OR⁶ oder
-O-(C₁-C₄-Alkylen)-CO-O-(C₁-C₄-Alkylen)-CO-N(R⁷)R⁸,
R⁶ C₁-C₄-Alkyl, C₂-C₄-Alkenyl oder C₃-C₄-Alkinyl,
R⁷ C₁-C₄-Alkyl oder (C₁-C₄-Alkoxy)carbonyl-C₁-C₄-alkyl und
R⁸ Wasserstoff oder C₁-C₄-Alkyl.

3. Verwendung der substituierten 2-Phenylpyridine der Formel I und der landwirtschaftlich brauchbaren Salze von I, gemäß Anspruch 1, als Herbizide oder zur Desikkation und/oder Defoliation von Pflanzen.

4. Herbizides Mittel, enthaltend eine herbizid wirksame Menge mindestens eines substituierten 2-Phenylpyridins der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

5. Mittel zur Desikkation und/oder Defoliation von Pflanzen, enthaltend eine desikkant und/oder defoliant wirksame Menge mindestens eines substituierten 2-Phenylpyridins der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

6. Verfahren zur Herstellung von herbizid wirksamen Mitteln, **dadurch gekennzeichnet, daß** man eine herbizid wirksame Menge mindestens eines substituierten 2-Phenylpyridins der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff mischt.

7. Verfahren zur Herstellung von desikkant und/oder defoliant wirksamen Mitteln, **dadurch gekennzeichnet, daß** man eine desikkant und/oder defoliant wirksame Menge mindestens eines substituierten 2-Phenylpyridins der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff mischt.

8. Verfahren zur Bekämpfung von unerwünschtem Pflanzenwuchs, **dadurch gekennzeichnet, daß** man eine herbizid wirksame Menge mindestens eines substituierten 2-Phenylpyridins der Formel I oder eines landwirtschaftlich brauchbaren Salzes von I, gemäß Anspruch 1, auf Pflanzen, deren Lebensraum oder auf Saatgut einwirken läßt.

9. Verfahren zur Desikkation und/oder Defoliation von Pflanzen, **dadurch gekennzeichnet, daß** man eine desikkant und/oder defoliant wirksame Menge mindestens eines substituierten 2-Phenylpyridins der Formel I oder eines landwirtschaftlich brauchbaren Salzes von I, gemäß Arspruch 1, auf Pflanzen einwirken läßt.

10. Verfahren nach Anspruch 9, **dadurch gekennzeichnet, daß** man Baumwolle behandelt.

11. Verfahren zur Herstellung von substituierten 2-Phenylpyridinen der Formel I gemäß Anspruch 1, **dadurch gekennzeichnet, daß** man in Gegenwart einer Base
a) ein Säurechlorid der Formel IIa 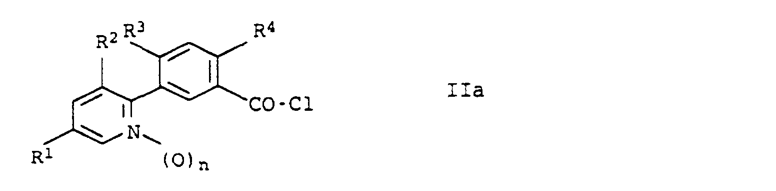 mit einer Hydroxycarbonsäure oder einem Derivat hiervon der Formel IIIa oder IIIb
HO-(C₁-C₄-Alkylen)-CO-OR⁶ IIIa
HO-(C₁-C₄-Alkylen)-CO-N(R⁷)R⁸ IIIb
b) ein Säurechlorid der Formel IIb 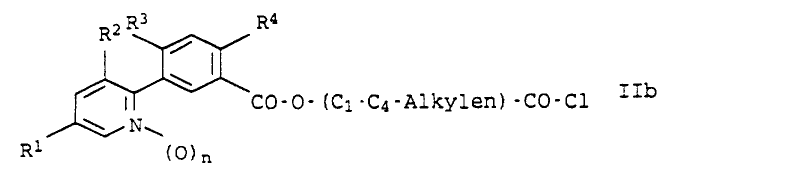 mit einem Alkohol IIIc oder Amin IIId
HO-R⁶ IIIc HN(R⁷)R⁸ IIId
c) ein Säurechlorid der Formel IIc 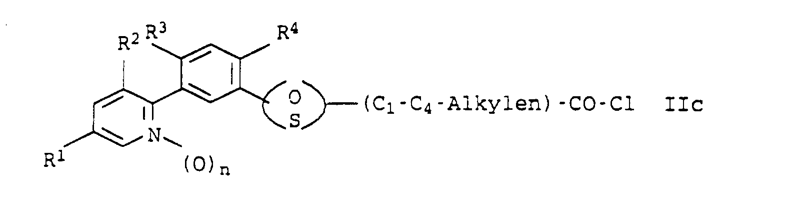 mit einer Hydroxycarbonsäure oder einem Derivat hiervon der Formel IIIa oder IIIb umsetzt.

12. Säurechloride der Formel IIb 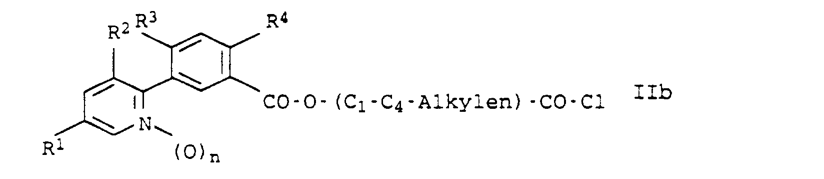 in der die Variablen folgende Bedeutung haben:
n 0 oder 1;
R¹ Halogen oder C₁-C₄-Halogenalkyl;
R², R³ jeweils Wasserstoff oder Halogen;
R⁴ Cyano oder Halogen.

13. Verfahren zur Herstellung der Säurechloride der Formel IIb gemäß Anspruch 12, **dadurch gekennzeichnet, daß** man ein Säurechlorid der Formel IIa mit einer Hydroxycarbonsäure HO-(C₁-C₄-Alkylen)-CO-OH (IV) oder einem Salz davon umsetzt und das Verfahrensprodukt anschließend auf an sich bekannte Weise chloriert.

14. Verfahren zur Herstellung von substituierten 2-Phenylpyridinen der Formel I gemäß Anspruch 1, wobei n für 1 steht und der Substituent R⁵ keine Schwefelbrücke enthält, **dadurch gekennzeichnet, daß** man die entsprechenden substituierten 2-Phenylpyridine, bei denen n Null bedeutet, auf an sich bekannte Weise in einem inerten Lösungs- oder Verdünnungsmittel oxidiert.

15. Verfahren zur Herstellung von substituierten 2-Phenylpyridinen der Formel I gemäß Anspruch 1, wobei R⁵ -O-(C₁-C₄-Alkylen)-CO-O-(C₁-C₄-Alkylen)-CO-OR⁶ oder -O-(C₁-C₄-Alkylen)-CO-O-(C₁-C₄-Alkylen)-CO-N(R⁷)R⁸ bedeutet,
**dadurch gekennzeichnet, daß** man ein 3-Pyridylphenol der Formel VI 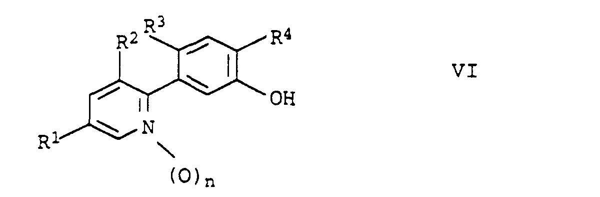 auf an sich bekannte Weise in einem inerten Lösungs- oder Verdünnungsmittel, in Gegenwart einer Base, mit einem Elektrophil der Formel VII
L-(C₁-C₄-Alkylen)-CO-O-(C₁-C₄-Alkylen)-CO-OR⁶ (VII)
oder der Formel VIII
L-(C₁-C₄-Alkylen)-CO-O-(C₁-C₄-Alkylen)-CO-N(R⁷)R⁸ (VIII)
wobei L jeweils für Chlor, Brom, Jod, Methylsulfonyloxy, Trifluormethylsulfonyloxy, Phenylsulfonyloxy oder p-Toluolsulfonyloxy steht, umsetzt.

16. Verfahren zur Herstellung von substituierten 2-Phenylpyridinen der Formel I gemäß Anspruch 1, wobei R⁵ -S-(C₁-C₄-Alkylen)-CO-O-(C₁-C₄-Alkylen)-CO-OR⁶ oder -S-(C₁-C₄-Alkylen)-CO-O-(C₁-C₄-Alkylen)-CO-N(R⁷)R⁸ bedeutet, **dadurch gekennzeichnet, daß** man ein 3-Pyridylthiophenol der Formel IX 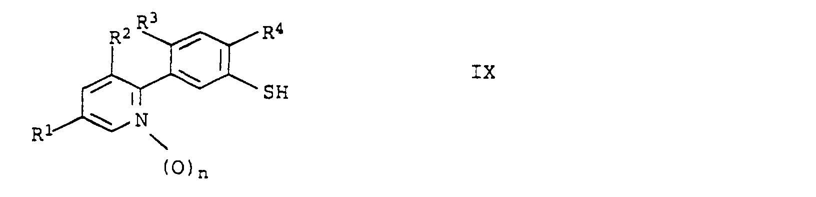 auf an sich bekannte Weise in einem inerten Lösungs- oder Verdünnungsmittel, in Gegenwart einer Base, mit einem Elektrophil der Formel VII
L-(C₁-C₄-Alkylen)-CO-O-(C₁-C₄-Alkylen)-CO-OR⁶ (VII)
oder der Formel VIII
L-(C₁-C₄-Alkylen)-CO-O-(C₁-C₄-Alkylen)-CO-N(R⁷)R⁸ (VIII)
wobei L jeweils für Chlor, Brom, Jod, Methylsulfonyloxy, Trifluormethylsulfonyloxy, Phenylsulfonyloxy oder p-Toluolsulfonyloxy steht, umsetzt.

## Claims

1. A substituted 2-phenylpyridine of the general formula I 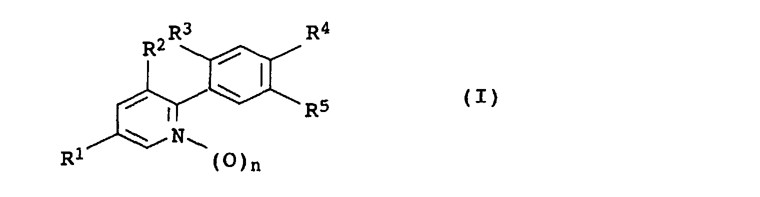 where the variables have the following meanings:
n is 0 or 1;
R¹ is halogen or C₁-C₄-haloalkyl;
R² and R³ are in each case hydrogen or halogen;
R⁴ is cyano or halogen;
R⁵ is -CO-O-(C₁-C₄-alkylene)-CO-OR⁶,
-CO-O-(C₁-C₄-alkylene)-CO-N(R⁷)R⁸,
-O-(C₁-C₄-alkylene)-CO-O-(C₁-C₄-alkylene)-CO-OR⁶,
-O-(C₁-C₄-alkylene)-CO-O-(C₁-C₄-alkylene)-CO-N(R⁷)R⁸,-S-(C₁-C₄-alkylene)-CO-O-(C₁-C₄-alkylene)-CO-OR⁶ or -S-(C₁-C₄-alkylene)-CO-O-(C₁-C₄-alkylene)-CO-N(R⁷)R⁸, where
R⁶ is hydrogen, C₁-C₄-alkyl, C₁-C₄-alkoxy-C₁-C₄-alkyl, C₂-C₄-alkenyl or C₃-C₄-alkynyl,
R⁷ is hydrogen, C₁-C₄-alkyl, carboxyl-C₁-C₄-alkyl, (C₁-C₄-alkoxy)carbonyl-C₁-C₄-alkyl and
R⁸ is hydrogen or C₁-C₄-alkyl,
or an agriculturally useful salt of a compound I where R⁶ = hydrogen.

2. A substituted 2-phenylpyridine of the formula I as claimed in claim 1, where the variables have the following meanings:
n is zero,
R¹ is chlorine or trifluoromethyl,
R² is chlorine,
R³ is hydrogen, fluorine or chlorine,
R⁴ is cyano or chlorine,
R⁵ is -CO-O-(C₁-C₄-alkylene)-CO-OR⁶,
-CO-O-(C₁-C₄-alkylene)-CO-N(R⁷)R⁸,
-O-(C₁-C₄-alkylene)-CO-O(C₁-C₄-alkylene)-CO-OR⁶ or
-O-(C₁-C₄-alkylene)-CO-O-(C₁-C₄-alkylene)-CO-N(R⁷)R⁸,
R⁶ is C₁-C₄-alkyl, C₂-C₄-alkenyl or C₃-C₄-alkynyl,
R⁷ is C₁-C₄-alkyl or (C₁-C₄-alkoxy)carbonyl-C₁-C₄-alkyl and
R⁸ is hydrogen or C₁-C₄-alkyl.

3. The use of a substituted 2-phenylpyridine of the formula I or of an agriculturally useful salt of I as claimed in claim 1 as a herbicide or for the desiccation and/or defoliation of plants.

4. A herbicidal composition comprising a herbicidally active amount of at least one substituted 2-phenylpyridine of the formula I or of an agriculturally useful salt of I as claimed in claim 1 and at least one inert liquid and/or solid carrier and, if desired, at least one surfactant.

5. A composition for the desiccation and/or defoliation of plants comprising such an amount of at least one substituted 2-phenylpyridine of the formula I or of an agriculturally useful salt of I as claimed in claim 1 that it acts as a desiccant and/or defoliant, and at least one inert liquid and/or solid carrier and, if desired, at least one surfactant.

6. A process for the preparation of a herbicidally active composition which comprises mixing a herbicidally active amount of at least one substituted 2-phenylpyridine of the formula I or of an agriculturally useful salt of I as claimed in claim 1 and at least one inert liquid and/or solid carrier and, if desired, at least one surfactant.

7. A process for the preparation of a composition having a desiccant and/or defoliant action, which comprises mixing such an amount of at least one substituted 2-phenylpyridine of the formula I or of an agriculturally useful salt of I as claimed in claim 1 that it acts as a desiccant and/or defoliant, and at least one inert liquid and/or solid carrier and, if desired, at least one surfactant.

8. A method of controlling undesirable vegetation, which comprises allowing a herbicidally active amount of at least one substituted 2-phenylpyridine of the formula I or of an agriculturally useful salt of I as claimed in claim 1 to act on plants, their environment or on seed.

9. A method for the desiccation and/or defoliation of plants, which comrises allowing such an amount of at least one substituted 2-phenylpyridine of the formula I or of an agriculturally useful salt of I as claimed in claim 1 that it acts as a desiccant and/or defoliant to act on plants.

10. A method as claimed in claim 9, which comprises the treatment of cotton.

11. A process for the preparation of a substituted 2-phenylpyridine of the formula I as claimed in claim 1, which comprises reacting, in the presence of a base,
a) an acid chloride of the formula IIa 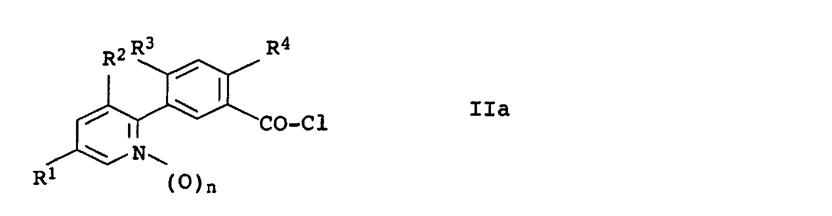 with a hydroxycarboxylic acid or one of its derivatives of the formula IIIa or IIIb
HO-(C₁-C₄-alkylene)-CO-OR⁶ IIIa
HO-(C₁-C₄-alkylene)-CO-N(R⁷)R⁸ IIIb
b) an acid chloride of the formula IIb 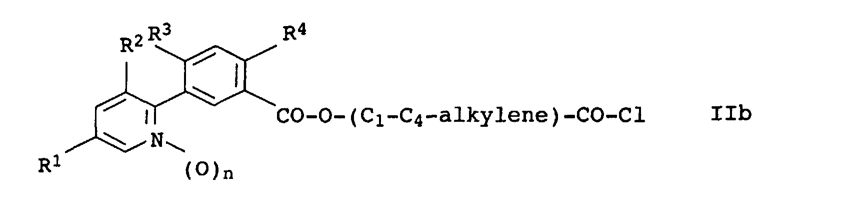 with an alcohol IIIc or amine IIId
HO-R⁶ IIIc HN(R⁷)R⁸ IIId
c) an acid chloride of the formula IIc 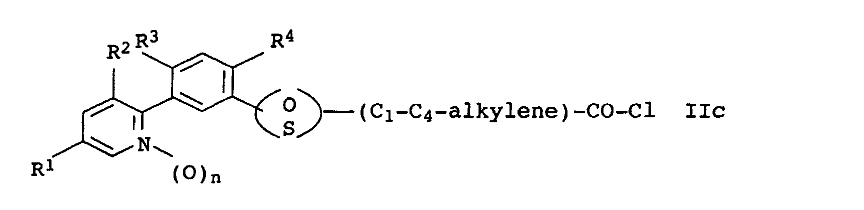 with a hydroxycarboxylic acid or one of its derivatives of the formula IIIa or IIIb.

12. An acid chloride of the formula IIb 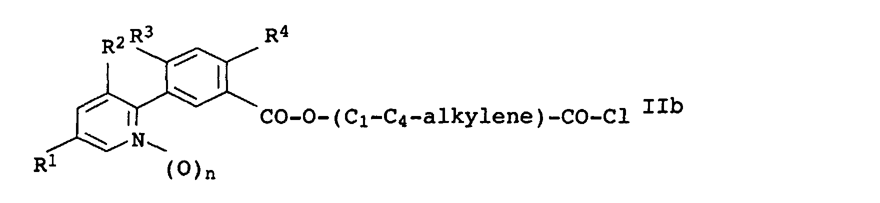 where the variables have the following meanings:
n is 0 or 1;
R¹ is halogen or C₁-C₄-haloalkyl;
R² and R³ are in each case hydrogen or halogen;
R⁴ is cyano or halogen.

13. A process for the preparation of an acid chloride of the formula IIb as claimed in claim 12, which comprises reacting an acid chloride of the formula IIa with a hydroxycarboxylic acid HO-(C₁-C₄-alkylene)-CO-OH (IV) or with a salt thereof and subsequently chlorinating the process product in a manner known per se.

14. A process for the preparation of a substituted 2-phenylpyridine of the formula I as claimed in claim 1 where n is 1 and the substituent R⁵ does not contain a sulfur bridge, which comprises oxidizing the corresponding substituted 2-phenylpyridines where n is zero in a manner known per se in an inert solvent or diluent.

15. A process for the preparation of a substituted 2-phenylpyridine of the formula I as claimed in claim 1, where R⁵ is -O-(C₁-C₄-alkylene)-CO-O-(C₁-C₄-alkylene)-CO-OR⁶ or -O-(C₁-C₄-alkylene)-CO-O-(C₁-C₄-alkylene)-CO-N(R⁷)R⁸, which comprises reacting a 3-pyridylphenol of the formula VI 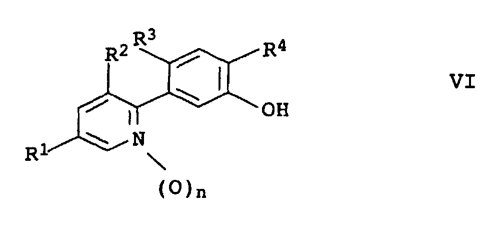 in a manner known per se in an inert solvent or diluent, in the presence of a base, with an electrophile of the formula VII
L-(C₁-C₄-alkylene)-CO-O-(C₁-C₄-alkylene)-CO-OR⁶ (VII)
or the formula VIII
L-(C₁-C₄-alkylene)-CO-O-(C₁-C₄-alkylene)-CO-N(R⁷)R⁸ (VIII)
where L is in each case chlorine, bromine, iodine, methylsulfonyloxy, trifluoromethylsulfonyloxy, phenylsulfonyloxy or p-toluenesulfonyloxy.

16. A process for the preparation of a substituted 2-phenylpyridine of the formula I as claimed in claim 1 where R⁵ is -S-(C₁-C₄-alkylene)-CO-O-(C₁-C₄-alkylene)-CO-OR⁶ or -S-(C₁-C₄-alkylene)-CO-O-(C₁-C₄-alkylene)-CO-N(R⁷)R⁸, which comprises reacting a 3-pyridylthiophenol of the formula IX 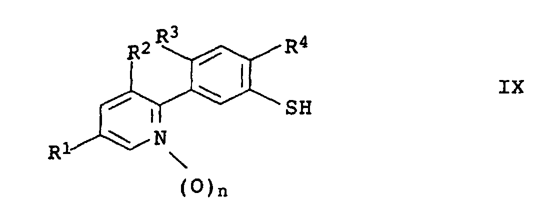 in a manner known per se in an inert solvent or diluent, in the presence of a base, with an electrophile of the formula VII
L-(C₁-C₄-alkylene)-CO-O-(C₁-C₄-alkylene)-CO-OR⁶ (VII)
or the formula VIII
L-(C₁-C₄-alkylene)-CO-O-(C₁-C₄-alkylene)-CO-N(R⁷)R⁸ (VIII)
where L is in each case chlorine, bromine, iodine, methylsulfonyloxy, trifluoromethylsulfonyloxy, phenylsulfonyloxy or p-toluenesulfonyloxy.

## Revendications

1. 2-phénylpyridines substituées de formule générale I 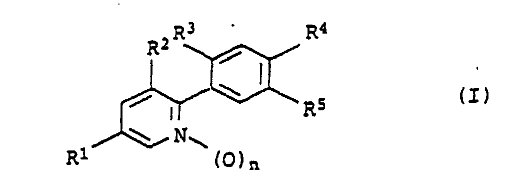 dans laquelle les symboles ont les significations suivantes :
n : 0 ou 1 ;
R¹ : un halogène ou un groupe halogénoalkyle en C1-C4;
R², R³ : chacun l'hydrogène ou un halogène ;
R⁴ : un groupe cyano ou un halogène ;
R⁵: un groupe -CO-O-(alkylène en C1-C4)-CO-OR⁶,
-CO-O-(alkylène en C1-C4)-CO-N(R⁷)R⁸,
-O-(alkylène en C1-C4)-CO-O-(alkylène en C1-C4)-CO-OR⁶,
-O-(alkylène en C1-C4)-CO-O-(alkylène en C1-C4)-CO-N(R⁷)R⁸,
-S-(alkylène en C1-C4)-CO-O-(alkylène en C1-C4)-CO-OR⁶ ou
-S-(alkylène en C1-C4)-CO-O-(alkylène en C1-C4)-CO-N(R⁷)R⁸,
dans lequel
R⁶ représente l'hydrogène, un groupe alkyle en C1-C4, (alcoxy en C1-C4)alkyle en C1-C4, alcényle en C2-C4 ou alcynyle en C3-C4,
R⁷ représente l' hydrogène, un groupe alkyle en C1-C4, carboxyl-alkyle en C1-C4, (alcoxy en C1-C4)carbonyl-alkyle en C1-C4 et
R⁸ représente l'hydrogène ou un groupe alkyle en C1-C4,
et les sels, aptes aux applications agricoles, des composés I pour lesquels R⁶ représente l'hydrogène.

2. 2-phénylpyridines substituées de formule I de la revendication 1 dans laquelle les symboles ont les significations suivantes :
n : 0,
R¹ : le chlore ou un groupe trifluorométhyle, R² : le chlore,
R³ : l'hydrogène, le fluor ou le chlore, R⁴ : un groupe cyano ou le chlore,
R⁵ : -CO-O-(alkylène en C1-C4)-CO-OR⁶,
-CO-O(alkylène en C1-C4)-CO-N(R⁷)R⁸,
-O-(alkylène en C1-C4)-CO-O(alkylène en C1-C4)-CO-OR⁶ ou
-O-(alkylène en C1-C4)-CO-O-(alkylène en C1-C4)-CO-N(R⁷)R⁸,
R⁶ : un groupe alkyle en C1-C4, alcényle en C2-C4 ou alcynyle en C3-C4,
R⁷ : un groupe alkyle en C1-C4 ou (alcoxy en C1-C4)carbonyl-alkyle en C1-C4 et R⁸ : l'hydrogène ou un groupe alkyle en C1-C4.

3. Utilisation des 2-phénylpyridines substituées de formule I et de leurs sels aptes aux applications agricoles selon revendication 1 en tant qu'herbicides ou pour la dessiccation et/ou la défoliation de végétaux.

4. Produit herbicide contenant une quantité herbicide efficace d'au moins une 2-phénylpyridine substituée de formule I ou d'un sel de I apte aux applications agricoles selon revendication 1 et au moins un véhicule liquide et/ou solide inerte, ainsi que, si on le désire, au moins une substance tensio-active.

5. Produit pour la dessiccation et/ou la défoliation de végétaux, contenant une quantité dessiccante et/ou défoliante efficace d'au moins une 2-phényl-pyridine substituée de formule I ou d'un sel de I apte aux applications agricoles selon revendication 1, et au moins un véhicule liquide et/ou solide inerte ainsi que, si on le désire, au moins une substance tensio-active.

6. Procédé pour la préparation de produits à activité herbicide, **caractérisé par le fait que** l'on mélange une quantité herbicide efficace d'au moins une 2-phénylpyridine substituée de formule I ou d'un sel apte aux applications agricoles de I selon revendication 1 et au moins un véhicule liquide et/ou solide inerte ainsi que, si on le désire, au moins une substance tensio-active.

7. Procédé pour la préparation de produits à activité dessiccante et/ou défoliante, **caractérisé par le fait que** l'on mélange une quantité dessiccante et/ou défoliante efficace d'au moins une 2-phénylpyridine substituée de formule I ou d'un sel apte aux applications agricoles de I selon revendication 1 et au moins un véhicule liquide et/ou solide inerte ainsi que, si on le désire, au moins une substance tensio-active.

8. Procédé pour combattre les croissances de végétaux indésirables, **caractérisé par le fait que** l'on fait agir une quantité herbicide efficace d'au moins une 2-phényl-pyridine substituée de formule I ou d'un sel de I apte aux applications agricoles selon revendication 1 sur les végétaux, leur habitat ou sur les semences.

9. Procédé pour la dessiccation et/ou la défoliation de végétaux, **caractérisé par le fait que** l'on fait agir une quantité dessiccante et/ou défoliante efficace d'au moins une 2-phénylpyridine substituée de formule I ou d'un sel apte aux applications agricoles de I selon revendication 1 sur les végétaux.

10. Procédé selon revendication 9, **caractérisé par le fait que** l'on traite le coton.

11. Procédé pour la préparation des 2-phénylpyridines substituées de formule I de la revendication 1, **caractérisé par le fait que** l'on fait réagir en présence d'une base
a) un chlorure d'acide de formule IIa 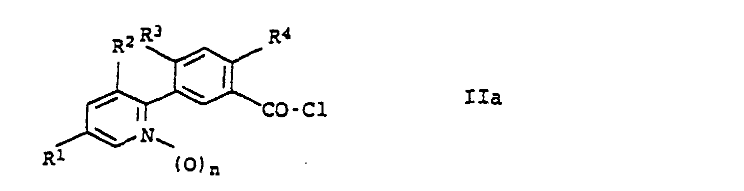 avec un acide hydroxycarboxylique ou un dérivé d'acide hydroxycarboxylique de formule IIIa ou IIIb
HO-(alkylène en C1-C4)-CO-OR⁶ IIIa
HO-(alkylène en C1-C4)-CO-N(R⁷)R⁸ IIIb
b) un chlorure d'acide de formule IIb 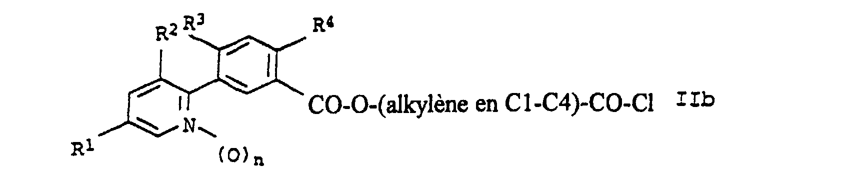 avec un alcool IIIc ou une amine IIId
HO-R⁶ IIIc HN(R⁷)R⁸ IIId
b) un chlorure d'acide de formule IIc 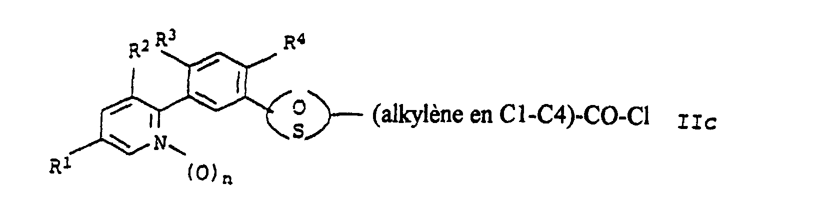 avec un acide hydroxycarboxylique ou un dérivé d'acide hydroxycarboxylique de formule IIIa ou IIIb.

12. Chlorures d'acides de formule IIb 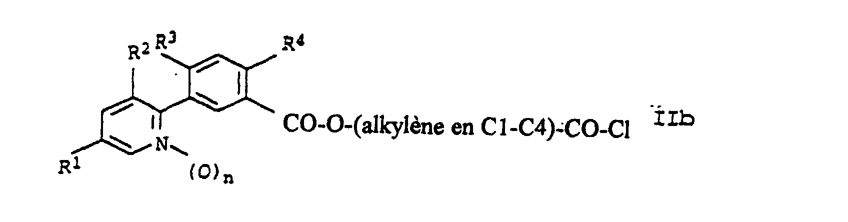 dans laquelle les symboles ont les significations suivantes :
n: 0 ou 1;
R^{1:} un halogène ou un groupe halogénoalkyle en C1-C4;
R², R³ : chacun l'hydrogène ou un halogène ;
R^{4:} un groupe cyano ou un halogène.

13. Procédé pour la préparation des chlorures d'acides de formule IIb de la revendication 12, **caractérisé par le fait que** l'on fait réagir un chlorure d'acide de formule IIa avec un acide hydroxycarboxylique HO-(allrylène en C1-C4)-CO-OH (IV) ou l'un de ses sels et on chlore ensuite le produit obtenu de manière connue en soi.

14. Procédé pour la préparation des 2-phénylpyridines substituées de formule I de la revendication 1 dans laquelle n est égal à 1 et le substituant R⁵ ne contient pas de pont sulfuré, **caractérisé par le fait que** l'on oxyde de manière connue en soi, dans un solvant ou diluant inerte, les 2-phénylpyridines substituées correspondantes pour lesquelles n est égal à 0.

15. Procédé pour la préparation des 2-phénylpyridines substituées de formule I de la revendication 1 dans laquelle R⁵ représente
-O-(alkylène en C1-C4)-CO-O-(alkylène en C1-C4)-CO-OR⁶ ou
-O-(alkylène en C1-C4)-CO-O-(alkylène en C1-C4)-CO-N(R⁷)R⁸, **caractérisé par le fait que** l'on fait réagir un 3-pyridylphénol de formule VI 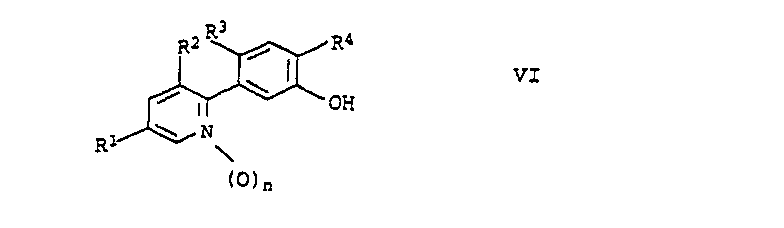 de manière connue en soi, dans un solvant ou diluant inerte et en présence d'une base, avec un réactif électrophile de formule VII
L-(alkylène en C1-C4)-CO-O-(alkylène en C1-C4)-CO-OR⁶ (VII)
ou de formule VIII
L-(alkylène en C1-C4)-CO-O-(alkylène en C1-C4)-CO-N(R⁷)R⁸ (VIII)
L représentant dans chaque cas le chlore, le brome, l'iode, un groupe méthylsulfonyloxy, trifluorométhylsulfonyloxy, phénylsulfonyloxy ou p-toluènesulfonyloxy.

16. Procédé pour la préparation des 2-phénylpyridines substituées de formule I de la revendication 1 dans laquelle R⁵ représente
-S-(alkylène en C1-C4)-CO-O-(alkylène en C1-C4)-CO-OR⁶ ou
-S-(alkylène en C1-C4)-CO-O-(alkylène en C1-C4)-CO-N(R⁷)R⁸ **caractérisé par le fait que** l'on fait réagir un 3-pyridylthiophénol de formule IX 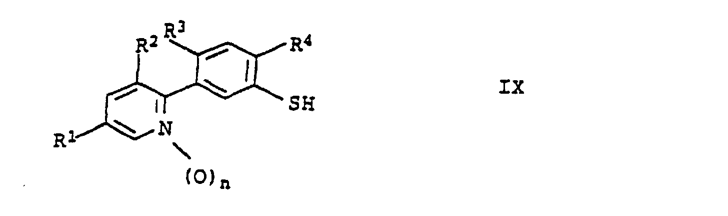 de manière connue en soi, dans un solvant ou diluant inerte et en présence d'une base, avec un réactif électrophile de formule VII
L-(alkylène en C1-C4)-CO-O-(alkylène en C1-C4)-CO-OR⁶ (VII)
ou de formule VIII
L-(alkylène en C1-C4)-CO-O-(alkylène en C1-C4)-CO-N(R⁷)R⁸ (VIII)
L représentant dans chaque cas le chlore, le brome, l'iode, un groupe méthylsulfonyloxy, trifluorométhylsulfonyloxy, phénylsulfonyloxy ou p-toluènesulfonyloxy.
